Europäisches Patentamt

European Patent Office

Office européen des brevets

(19)

(11) Publication number: 0 340 170 A2

# EUROPEAN PATENT APPLICATION

(12)

(21) Application number: 89810297.5

(22) Date of filing: 20.04.89

(51) Int. Cl.⁴: **C 12 N 15/00**
C 12 P 21/02, C 12 N 1/18
//(C12P21/02,C12R1:865)

(30) Priority: 26.04.88 GB 8809860

(43) Date of publication of application:
02.11.89 Bulletin 89/44

(84) Designated Contracting States:
AT BE CH DE ES FR GB GR IT LI LU NL SE

(71) Applicant: CIBA-GEIGY AG
Klybeckstrasse 141
CH-4002 Basel (CH)

UCP GEN-PHARMA AG
Solothurnstrasse 24
CH-3422 Kirchberg (CH)

(72) Inventor: Meyhack, Bernd, Dr.
Höhenweg 9
CH-4312 Magden (CH)

Pridmore, Raymond, Dr.
Chemin de la Résidence 7
CH-1009 Pully (CH)

Grossenbacher, Hugo, Dr.
Martin-Distellstrasse 79
CH-4600 Olten (CH)

Heim, Jutta, Dr.
Zelgliring 17
CH-4433 Ramlinsburg (CH)

The applicant has filed a statement in accordance with Rule 28 (4) EPC (issue of a sample only to an expert). Accession number(s) of the deposit(s): DSM 4413, DSM 4414, DSM 4473

(54) Process for the production of polypeptides.

(57) A novel process for the production of desulphatohirudin compounds including the use of genetically engineered yeast strains is provided. The invention concerns also novel hybrid vectors comprising a hirudin expression cassette, yeast strains containing such hybrid vectors, novel desulphatohirudin compounds and methods for the production thereof.

Fig. 4: Construction of plasmid pCP3L/GAPFL-YHIR

EP 0 340 170 A2

## Description

### Process for the production of polypeptides

The invention pertains to the field of recombinant DNA technology and concerns a method for the preparation of polypeptides with an anticoagulant action, more especially desulphatohirudins, with the aid of genetically engineered yeast cells, said genetically engineered yeast cells, hybrid vectors carrying the genes for said desulphatohirudins and methods for the preparation of said yeast cells and said hybrid vectors.

Hirudin is an anticoagulant agent that occurs naturally in leeches (Hirudo medicinalis). Hirudin is not a single polypeptide species but a class of equally acting polypeptides consisting of at least four representatives designated hirudin variant 1 (HV1), hirudin variant 2 (HV2) (cf. European Patent Application No. 158 564), hirudin variant PA (cf. PCT-Application No. 86/03493) and "des-(Val)$_2$-hirudin" (cf. European Patent Application No. 158 986). The variants differ in structure from each other by a number of amino acids (especially, the N-terminal sequence of HV1 is Val-Val-Tyr, that of HV2 and of PA is Ile-Thr-Tyr and that of "des-(Val)$_2$-hirudin" is Thr-Tyr) but have an accumulation of hydrophobic amino acids at the N-terminus and of polar amino acids at the C-terminus, a tyrosine residue (Tyr$^{63}$) present as sulphate monoester, three disulphide bridges and the anticoagulant activity in common.

Hirudin is the strongest thrombin inhibitor known and is characterised by a specific affinity to thrombin. Other enzymes of the blood coagulation cascade are not inhibited by hirudin. In contrast to heparin which is the preferred anticoagulant in conventional anticoagulation therapy, hirudin exerts its inhibiting action directly on thrombin and, unlike the former, does not act through antithrombin III. The only pharmacologically detectable effect of purified hirudin is the inhibition of blood coagulation and the prophylaxis of thrombosis. No effect on heart rate, respiration, blood pressure, thrombocyte count, fibrinogen and haemoglobin could be observed after intravenous administration of hirudin to dogs, even in high doses. In tests on rats, pigs and dogs, hirudin has proved effective in experimental thrombosis (induced either by stasis or by the injection of thrombin), in endotoxin shock, and also in DIC (disseminated intravascular coagulation). Whenever direct comparison tests have been carried out, hirudin has proved to be superior to heparin. Furthermore, hirudin has an extremely low toxicity, is non-antigenic and shows an almost complete clearance via the kidneys in a biologically active form.

Recently, cDNAs and synthetic genes coding for hirudin variants have been cloned and expressed in microbial hosts. Although the expression products lack the sulphate monoester group at Tyr$^{63}$ - and were therefore designated "desulphatohirudins" - they turned out to exhibit approximately the same biological activity as the natural sulphated hirudins. Desulphatohirudin variant HV1 has been expressed in Escherichia coli (European Patent Applications No. 158 564 and 168 342) and in Saccharomyces cerevisiae (European Patent Applications No. 168 342, 200 655, 225 633 and 252 854). Similarly, desulphatohirudin HV2 has been expressed in Escherichia coli (European Patent Applications No. 158 564) and in Saccharomyces cerevisiae (European Patent Application No. 200 655, PCT-Application No. 86/01224) and des-(Val)$_2$-desulphatohirudin has been expressed in Escherichia coli (European Patent Application No. 158 986).

Generally, expression efficiency and yields in hirudin compounds are higher when S. cerevisiae is chosen as the host microorganism. However, even with the available expression systems developed for S. cerevisiae the yields are relatively poor. In view of this, there is a need for improved methods which render possible the economic production of desulphatohirudins on a large scale. It is an object of the present invention to provide such methods.

Most strains of Saccharomyces cerevisiae carry a high copy number, self-replicating, extrachromosomal DNA element, designated as two-micron plasmid. The most striking structural features of the two-micron plasmid are two inverted repeats (IR1 and IR2) of 599 bp each dividing the plasmid in two DNA regions of different length. The homologous recombination between these two identical IR sequences results in the formation of two molecular isomers (form A and form B). Apart from the IR regions, the two-micron plasmid contains an origin of replication (ORI), four open reading frames (REP1, REP2, FLP, D) and a STB (or REP3) site. The FLP gene product is a site-specific recombinase which acts on the IR structures and is controlled by the REP1, REP2 and D gene products. The REP1 and REP2 gene products and the STB site are essential for the stable partitioning of the two-micron plasmids from mother to daughter cells.

Hybrid vectors devised for the expression of heterologous genes in yeast most frequently contain two-micron DNA. In general this two-micron DNA includes the origin of replication although this is not a basic condition for stable transformation because the two-micron DNA containing hybrid vectors can recombine with the endogenous two-micron plasmids via the IR sequences. The yeast hirudin expression vectors disclosed in European Patent Applications No. 168 342, 200 655, 225 633 and 252 854 contain two-micron DNA fragments including the origin of replication, an IR sequence and in some cases the STB site.

Surprisingly, it has now been found that the stability of transformants and the yield in desulphatohirudin compounds can be increased considerably when the expression vector comprises the complete two-micron DNA and the host yeast strain is devoid of endogenous two-micron plasmids.

Accordingly, the invention relates to a method for the production of desulphatohirudin compounds comprising culturing a yeast strain which is devoid of endogenous two-micron DNA and has been transformed with a hybrid vector comprising (1) a desulphatohirudin expression cassette consisting of a constitutive yeast promoter operably linked to a first DNA sequence encoding a signal peptide linked in the proper reading frame

2

to a second DNA sequence coding for a desulphatohirudin, and a DNA sequence containing yeast transcription termination signals, and (2) the complete two-micron DNA including intact REP1, REP2 and FLP genes, as well as intact ORI, STB, IR1 and IR2 sites, and optionally an intact D gene, and isolating said desulphatohirudins, and, if desired, separating a mixture of desulphatohirudin compounds obtained in the individual components.

The term "desulphatohirudin" is intended to embrace all desulphatohirudin compounds described in literature or obtainable from a transformed microorganism strain containing DNA which codes for a desulphatohirudin. Such desulphatohirudins are, for example, desulphatohirudin variant HV1, HV2, HV2 (modified), PA and des-(Val$_2$)-desulphatohirudin. It is to be understood that derivatives having hirudin activity (i.e. having a thrombin inhibiting action) are also covered by the term "desulphatohirudin". Such derivatives are especially C-terminally shortened desulphatohirudins, i.e. desulphatohirudins lacking one to seven, preferably one to four, amino acids at the C-terminus.

Preferred desulphatohirudins are those having the formulae

```
    X Tyr Thr Asp Cys Thr Glu Ser Gly Gln Asn Leu Cys

Leu Cys Glu Gly Ser Asn Val Cys Gly Gln Gly Asn Lys Cys .

Ile Leu Gly Ser Asp Gly Glu Lys Asn Gln Cys Val Thr Gly

Glu Gly Thr Pro Lys Pro Gln Ser His Asn Asp Gly Asp Phe

Glu Glu Ile Pro Glu Glu Tyr Leu Gln
```

(I),

in which X represents the dipeptide residue Val-Val- (HV1) or Thr (Des-(Val)$_2$-hirudin) and the derivatives lacking the C-terminal amino acid Gln, the C-terminal dipeptide -Leu-Gln, the C-terminal tripeptide -Tyr-Leu-Gln or the C-terminal tetrapeptide -Glu-Tyr-Leu-Gln, or

```
 Y₁ Tyr Thr Asp Cys Thr Glu Ser Gly Gln Asn Leu Cys

Leu Cys Glu Gly Ser Asn Val Cys Gly Lys Gly Asn Lys Cys

Ile Leu Gly Ser Asn Gly Lys Gly Asn Gln Cys Val Thr Gly

Glu Gly Thr Pro Y₂  Pro Glu Ser His Asn Asn Gly Asp Phe

Glu Glu Ile Pro Glu Glu Tyr Leu Gln
```

(II)

in which Y$_1$ represents the N-terminal dipeptide residue Ile-Thr- and Y$_2$ represents Asn (HV2) or Lys, or Y$_1$ represents Val-Val and Y$_2$ is Asn or Lys (HV2 modified), or

```
Ile Thr Tyr Thr Asp Cys Thr Glu Ser Gly Gln Asn Leu Cys

Leu Cys Glu Gly Ser Asn Val Cys Gly Lys Gly Asn Lys Cys

Ile Leu Gly Ser Gln Gly Lys Asp Asn Gln Cys Val Thr Gly

Glu Gly Thr Pro Lys Pro Gln Ser His Asn Gln Gly Asp Phe

Glu Pro Ile Pro Glu Asp Ala Tyr Asp Glu
```

(III)

(PA). The most preferred desulphatohirudin compound is that of formula I in which X represents the dipeptide residue Val-Val-.

The yeast host strains and the constituents of the hybrid vectors are those specified below.

The transformed yeast strains are cultured using methods known in the art.

Thus, the transformed yeast strains according to the invention are cultured in a liquid medium containing assimilable sources of carbon, nitrogen and inorganic salts.

Various carbon sources are usable. Example of preferred carbon sources are assimilable carbohydrates, such as glucose, maltose, mannitol, fructose or lactose, or an acetate such as sodium acetate, which can be used either alone or in suitable mixtures. Suitable nitrogen sources include, for example, amino acids, such as casamino acids, peptides and proteins and their degradation products, such as tryptone, peptone or meat extracts, furthermore yeast extract, malt extract, corn steep liquor, as well as ammonium salts, such as ammonium chloride, sulphate or nitrate which can be used either alone or in suitable mixtures. Inorganic salts which may be used include, for example, sulphates, chlorides, phosphates and carbonates of sodium, potassium, magnesium and calcium. Additionally, the nutrient medium may also contain growth promoting substances. Substances which promote growth include, for example, trace elements, such as iron, zinc, manganese and the like, or individual amino acids.

Hybrid vectors comprising the complete two-micron DNA (including a functional origin of replication) are stably maintained within strains of Saccharomyces cerevisiae which are devoid of endogenous two-micron plasmids (so-called cir° strains) so that the cultivation can be carried out under non-selective growth conditions, i.e. in a complex medium.

The cultivation is carried out by employing conventional techniques. The culturing conditions, such as temperature, pH of the medium and fermentation time are selected in such a way that maximal levels of desulphatohirudin are produced. A chosen yeast strain is preferably grown under aerobic conditions in submerged culture with shaking or stirring at a temperature of about 25° to 35°C, preferably at about 28°C, at a pH value of from 4 to 7, for example at approximately pH 5, and for at least 1 to 3 days, preferably as long as satisfactory yields of desulphatohirudin are obtained.

Irrespective of the yeast strain, promoter and signal peptide used, most of the produced desulphatohirudin is secreted into the culture medium whereas only a minor part remains cell associated. The precise ratio (secreted compounds/cell associated compounds) depends on the fermentation conditions and the recovery process applied. In general it amounts to about or more than 8:1. Accordingly, secreted desulphatohirudin is always strongly dominating.

The desulphatohirudin can be isolated from the culture medium by conventional means. For example, the first step consists usually in separating the cells from the culture fluid by means of centrifugation. The resulting supernatant can be enriched for desulphatohirudin by treatment with polyethyleneimine so as to remove most of the non-proteinaceous material, and precipitation of the proteins by saturating the solution with ammonium sulphate. Host proteins, if present, can also be precipitated by means of acidification with acetic acid (for example 0.1 %, pH 4-5). A further enrichment of desulphatohirudin can be achieved by extracting the acetic acid supernatant with n-butanol. Other purification steps include, for example, desalination, chromatographic processes, such as ion exchange chromatography, gel filtration chromatography, partition chromatography, HPLC, reversed phase HPLC and the like. The separation of the constituents of the mixture is also effected by dialysis, according to charge by means of gel electrophoresis or carrier-free electrophoresis, according to molecular size by means of a suitable Sephadex column, by affinity chromatography, for example with antibodies, especially monoclonal antibodies, or with thrombin coupled to a suitable carrier for affinity chromatography, or by other processes, especially those known from the literature.

If it is desired to isolate any additional desulphatohirudin which is cell associated, i.e. which has accumulated intracellularly or in the periplasmic space, some supplementary purification steps are required. Thus, in case the desulphatohirudin has accumulated within the cells, the first step for the recovery thereof consists in liberating it from the cell interior. In most procedure the cell wall is first removed by enzymatic digestion with glucosidases (infra). Subsequently, the resulting speroplasts are treated with detergents, such as Triton. Alternatively, mechanical forces, such as shearing forces (for example X-press, French-press) or shaking with glass beads, are suitable for breaking cells. In the case where the desulphatohirudin is secreted by the host cells into the periplasmic space, a simplified protocol can be used: The desulphatohirudin is recovered without cell lysis by enzymatic removal of the cell wall or by treatment with chemical agents, e.g. thiol reagents or EDTA, which give rise to cell wall damages permitting the product to be released.

A mixture of desulphatohirudins obtained can be separated into the individual components by applying conventional means, such as chromatographic methods, for example reversed phase HPLC, ion exchange chromatography, gelfiltration chromatography and hydrophobic interaction chromatography.

The test with anti-hirudin or anti-desulphatohirudin antibodies (for example, monoclonal antibodies obtainable from hydridoma cells), the thrombin test [M.U. Bergmeyer (ed.), Methods in Enzymatic Analysis, Vol. II, p. 314-316, Verlag Chemie, Weinheim (FRG) 1983] or the blood coagulation test [F. Markwardt et al., Thromb. Haemost. 47, 226 (1982)] can be used to detect the hirudin activity.

The transformed yeast host cells according to the invention can be prepared by recombinant DNA techniques comprising the steps of
- preparing a hybrid vector comprising (1) a desulphatohirudin expression cassette consisting of a constitutive yeast promoter operably linked to a first DNA sequence encoding a signal peptide linked in the proper reading frame to a second DNA sequence coding for a desulphatohirudin, and a DNA sequence containing yeast transcription termination signals, and (2) the complete two-micron DNA including intact REP1, REP2 and FLP genes, as well as intact ORI, STB, IR1 and IR2 sites, and optionally an intact D gene,
- preparing a yeast strain which is devoid of endogenous two-micron DNA,
- transforming the cir° strain obtained with said hybrid vector,

4

- and selecting transformed yeast cells from untransformed yeast cells.

Expression vectors

The invention concerns a yeast hybrid vector comprising (1) a desulphatohirudin expression cassette consisting of a constitutive yeast promoter operably linked to a first DNA sequence encoding a signal peptide linked in the proper reading frame to a second DNA sequence coding for a desulphatohirudin, and a DNA sequence containing yeast transcription termination signals, and (2) the complete two-micron DNA including intact REP1, REP2 and FLP genes, as well as intact ORI, STB, IR1 and IR2 sites and a method for the production thereof.

The constitutive yeast promoter is preferably derived from a highly expressed yeast gene, such as a gene encoding a glycolytic enzyme, such as the promoter of the enolase, glyceraldehyde-3-phosphate dehydrogenase (GAPDH), 3-phosphoglycerate kinase (PGK), hexokinase, pyruvate decarboxylase, phosphofructokinase, glucose-6-phosphate isomerase, 3-phosphoglycerate mutase, pyruvate kinase, triosephosphate isomerase, phosphoglucose isomerase and glucokinase gene, furthermore the ADHI or TRPI promoter and a shortened acid phosphatase PHO5 promoter which has been deprived of its upstream activation sites. Especially preferred is the GAPDH promoter and functional fragments thereof starting at nucleotide between -550 and -180, in particular at nucleotide -540, -263 or -198, and ending at nucleotide -5 of the GAPDH gene, and shortened constitutive PHO5 promoters starting at nucleotide between -200 and -150, in particular at -173, and ending at nucleotide -9 of the PHO5 gene.

The DNA sequence encoding a signal peptide ("signal sequence") is preferably derived from a yeast gene coding for a polypeptide which is ordinarily secreted. The hirudin signal sequence obtainable from leech genome DNA can also be chosen. Yeast signal sequences are, for example, the signal and prepro sequences of the yeast invertase, $\alpha$-factor, pheromone peptidase (KEX1), "killer toxin" and repressible acid phosphatase (PHO5) genes and the glucoamylase signal sequence from Aspergillus awamori. Alternatively, fused signal sequences may be constructed by ligating part of the signal sequence (if present) of the gene naturally linked to the promoter used (for example PHO5), with part of the hirudin signal sequence. Those combinations are favoured which allow a precise cleavage between the signal sequence and the desulphatohirudin amino acid sequence. Additional sequences, such as pro- or spacer-sequences which may or may not carry specific processing signals can also be included in the constructions to facilitate accurate processing of precursor molecules. Alternatively, fused proteins can be generated containing internal processing signals which allow proper maturation in vivo or in vitro. For example, the processing signals contain a Lys-Arg residue, which is recognized by a yeast endopeptidase located in the Golgi membranes. The preferred signal sequences according to the present invention are those of the yeast PHO5 gene coding for a signal peptide having the formula

Met Phe Lys Ser Val Val Tyr Ser Ile Leu Ala Ala Ser Leu Ala Asn Ala,

and of the yeast invertase gene coding for a signal peptide having the formula

Met Leu Leu Gln Ala Phe Leu Phe Leu Leu Ala Gly Phe Ala Ala Lys Ile Ser Ala.

The DNA sequence coding for desulphatohirudin can be isolated from genomic leech DNA or a double-stranded desulphatohirudin DNA (desulphatohirudin ds cDNA) is produced complementary to desulphatohirudin mRNA, or a gene coding for the amino acid sequence of desulphatohirudin is produced by means of chemical and enzymatic processes, in a manner known per se.

A DNA sequence containing yeast transcription termination signals is preferably the 3′ flanking sequence of a yeast gene which contains proper signals for transcription termination and polyadenylation. Suitable 3′ flanking sequences are for example those of the yeast gene naturally linked to the promoter used. The preferred flanking sequence is that of the yeast PHO5 gene.

The yeast promoter, the DNA sequence coding for the signal peptide, the DNA sequence coding for desulphatohirudin and the DNA sequence containing yeast transcription termination signals are operably linked to each other, i.e. they are juxtaposed in such a manner that their normal functions are maintained. The array is such that the promoter effects proper expression of the signal sequence-desulphatohirudin gene complex, the transcription termination signals effect proper termination of transcription and polyadenylation and the signal sequence is linked in the proper reading frame to the desulphatohirudin gene in such a manner that the last codon of the signal sequence is directly linked to the first codon of the gene for desulphatohirudin and secretion of desulphatohirudin occurs. If the promoter and the signal sequence are derived from different genes, the promoter is preferably joined to the signal sequence between the major mRNA start and the ATG of the gene naturally linked to the promoter. The signal sequence should have its own ATG for translation initiation. The junction of these sequences may be effected by means of synthetic oligodeoxynucleotide linkers carrying the recognition sequence of an endonuclease.

The hybrid vectors according to the invention contain the complete two-micron DNA in an uninterrupted form, i.e. two-micron DNA is cleaved once with a restriction endonuclease, the linearised DNA is linked with the other components of the vector prior to recircularization. The restriction site is chosen such that normal function of the REP1, REP2 and FLP genes and of the ORI, STB, IR1 and IR2 sites is maintained. Optionally, the restriction site is chosen such that the D gene too is kept intact. Preferred restriction sites are the unique PstI located within the D gene and the unique HpaI and SnaBI sites located outside all of said genes and sites.

Preferably, the hybrid vectors according to the invention include one or more, especially one or two, selective genetic markers for yeast and such a marker and an origin of replication for a bacterial host,

especially <u>Escherichia coli.</u>

As to the selective gene markers for yeast, any marker gene can be used which facilitates the selection for transformants due to the phenotypic expression of the marker gene. Suitable markers for yeast are, for example, those expressing antibiotic resistance or, in the case of auxotrophic yeast mutants, genes which complement host lesions. Corresponding genes confer, for example, resistance to the antibiotics G418, hygromycin or bleomycin or provide for prototrophy in an auxotrophic yeast mutant, for example the <u>URA3</u>, <u>LEU2</u>, <u>LYS2</u> or <u>TRP1</u> gene.

As the amplification of the hybrid vectors is conveniently done in <u>E. coli</u>, an <u>E. coli</u> genetic marker and an <u>E. coli</u> replication origin are included advantageously. These can be obtained from <u>E. coli</u> plasmids, such as pBR322 or a pUC plasmid, for example pUC18 or pUC19, which contain both <u>E. coli</u> replication origin and <u>E. coli</u> genetic marker conferring resistance to antibiotics, such as ampicillin.

The hybrid vectors according to the invention can be prepared by methods known in the art. The process comprises cleaving two-micron plasmid DNA with a restriction endonuclease such that the normal function of the REP1, REP2 and FLP genes and of the ORI, STB, IR1 and IR2 sites is maintained, linking the linearized plasmid obtained to the desulphato hirudin expression cassette and optionally to DNA segments containing one or more selective genetic markers for yeast and a selective genetic marker and a replication origin for a bacterial host, and recircularizing the hybrid vector obtained.

<u>Preparation of two-micron free yeast strains</u>

Stability of the two-micron plasmid is given by three plasmid encoded functions. The REP1 and REP2 gene products are trans-acting proteins that are required for the stable partitioning of the two-micron plasmid. Of these two, REP1 is possibly the more important, in that the efficiency of partitioning is dependent on the gene dosage of the REP1 gene product [A. Cashmore et al., Mol. Gen. Genet. <u>203</u>, 154 (1986)]. These two proteins act through and on the STB (REP3) site, an important cis-acting element on the plasmid [M. Jayaram et al., Mol. Cell. Biol. (1985) 2466; B. Viet et al., Mol. Cell. Biol. (1985) 2190]. The following procedure is based on the presumption that curing of the two-micron plasmid by a second plasmid involves increasing the dosage of the STB site to titrate out the REP1 and REP2 proteins. This relative reduction of the REP1 and REP2 proteins would lead to an instability of the endogenous two-micron plasmid.

Preferably, the second plasmid used has a defect in or lacks the REP1 gene. An example of such a plasmid is pDP38 which apart from the REP1 gene lacks an inverted repeat (IR2). This makes its high copy number expression dependent on the complementation of REP1 protein by the endogenous two-micron plasmid. It contains two yeast selective markers: URA3, used in both high and low copy number situations, and dLEU2, applicable only in high copy number situations [E. Erhart et al., J. Bacteriol. (1968) 625].

A yeast strain which is Ura⁻ and Leu⁻ is transformed with plasmid pDP38 and selected for Ura⁺ colonies. The selection on uracile free plates (Ura selection) gives a much better transformation frequency than the selection on leucine free plates (Leu selection), as the URA3 gene is much better expressed than the defective dLEU2 gene. A single colony is selected and streaked onto a Leu selection plate which gives colonies of varying size and form. Some of the smallest colonies are restreaked onto Ura selection plates and replica-plated onto Leu selection plates. Those colonies are selected that can grow under Ura selection but only very slowly under Leu selection. Growth on Ura selection plates shows that the plasmid pDP38 is still present and that the merely slow growth under Leu selection is not due to the loss of this plasmid, and the failure of growth under Leu selection implies that pDP38 is not able to complement this marker. The latter fact can be explained in two ways: A. The LEU2 gene on pDP38 is mutated, or B: The plasmid cannot complement leu2 because it cannot raise its copy number, implying that the two-micron plasmid is not available (i.e. lost) to complement the REP1 gene product.

These two possibilities can be distinguished very easily. In the first place, the minimal growth seen with said colonies (as against the absolute zero growth of cells without pDP38) shows that some LEU2 expression is present. The second point can be directly tested, as in the absence of the two-micron plasmid pDP38 will act only as an ARS type plasmid, i.e. it will be very unstable so that most of the colonies will lose it after a few generations. Accordingly, when a single colony is streaked onto a YPD plate, and single colonies taken and replica-plated onto uracile free plates, then only a few will grow under Ura selection. Non growing colonies are checked by hybridization for pUC and two-micron sequences. Colonies which show no hybridization signals are free of plasmid pDP38 and of endogenous two-micron plasmids (cir°-strains).

The cir° strains according to the invention can also be prepared by other, though less advantageous, methods known from the art [see, for example, C. P. Hollenberg, Curr. Top. Microbiol. Immunol. <u>96</u>, 119 (1982)].

<u>Transformed yeast strains</u>

The invention concerns furthermore a yeast strain which is devoid of endogenous two-micron DNA and has been transformed with a hybrid vector comprising (1) a desulphatohirudin expression cassette consisting of a constitutive yeast promoter operably linked to a first DNA sequence encoding a signal peptide linked in the proper reading frame to a second DNA sequence coding for a desulphatohirudin, and a DNA sequence containing yeast transcription termination signals, and (2) the complete two-micron DNA including intact REP1, REP2 and FLP genes, as well as intact ORI, STB, IR1 and IR2 sites, and to a method for the production thereof.

Suitable yeast host strains include strains of <u>Saccharomyces cerevisiae</u> which have been cured of endogenous two-micron plasmids (see above).

The method for the production of said transformed yeast strain comprises transforming a yeast strain which is devoid of endogenous two-micron plasmids with said hybrid vector.

The transformation of yeast with the hybrid vectors according to the invention may be accomplished according to the method described by Hinnen et al. [Proc. Natl. Acad. Sci. USA <u>75</u>, 1929 (1978)]. This method can be divided into three steps:

(1) Removal of the yeast cell wall or parts thereof using various preparations of glucosidases, such as snail gut juices (e.g. Glusulase® or Helicase®) or enzym mixtures obtained from microorganisms (e.g. Zymolyase®) in osmotically stabilized solutions (e.g. 1 M sorbitol).

(2) Treatment of "naked" yeast cells (spheroplasts) with the DNA vector in the presence of PEG (polyethyleneglycol) and Ca$^{2+}$ ions.

(3) Regeneration of the cell wall and selection of the transformed cells in a solid layer of agar. This regeneration is conveniently done by embedding the spheroplasts into agar. For example, molten agar (about 50°C) is mixed with the spheroplasts. Upon cooling the solution to yeast growth temperatures (about 30°C), a solid layer is obtained. This agar layer is to prevent rapid diffusion and loss of essential macromolecules from the spheroplast and thereby facilitates regeneration of the cell wall. However, cell wall regeneration may also be obtained (although at lower efficiency) by plating the spheroplasts onto the surface of preformed agar layers.

Preferably, the regeneration agar is prepared in a way to allow regeneration and selection of transformed cells at the same time. Since yeast genes coding for enzymes of amino acid or nucleotide biosynthetic pathways are generally used as selective markers (supra), the generation is preferably performed in yeast minimal medium agar. If very high efficiencies of regeneration are required the following two step procedure is advantageous: (1) regeneration of the cell wall in a rich complex medium, and (2) selection of the transformed cells by replica plating the cell layer onto selective agar plates.

The invention concerns furthermore the new desulphatohirudin compounds obtainable by the process according to the invention.

The desulphatohirudin compounds obtainable by the process according to the invention can be used, analogously to natural hirudin, for the therapy and prophylaxis of thromboses, for acute shock therapy, for the therapy of consumption coagulopathies, and the like as described in European Patent Application No. 168 342.

The invention concerns especially the hybrid vectors, the transformed yeast strains, the methods for the production of said hybrid vectors and said transformed yeast strains and the method for the production of desulphatohirudin compounds, as described in the Examples.

## Brief description of the drawings

In the following experimental part various embodiments of the present invention are described with reference to the accompanying drawings in which:

Fig. 1 is a schematic diagram showing the <u>in vitro</u> synthesis of the hirudin HV1 gene including the <u>PH05</u> signal sequence with the preferred yeast codons. The 21 oligodeoxynucleotides used are indicated by numbered lines and dotted lines, respectively.

Fig. 2 schematically illustrates the construction of plasmid pDP33.

Fig. 3 schematically illustrates the construction of plasmids pDP34 and pDP38.

Fig. 4 schematically illustrates the construction of expression plasmid pDP34/GAPFL-YHIR.

Fig. 5 schematically illustrates the construction of expression plasmid pDP34/PH05(-173)-YHIR.

Fig. 6 schematically illustrates the construction of plasmid pDP92.

Fig. 7 is a schematic illustration of plasmid pDP96.

## Experimental Part

## Example 1: In vitro synthesis of the hirudin HV1 gene with preferred yeast codons

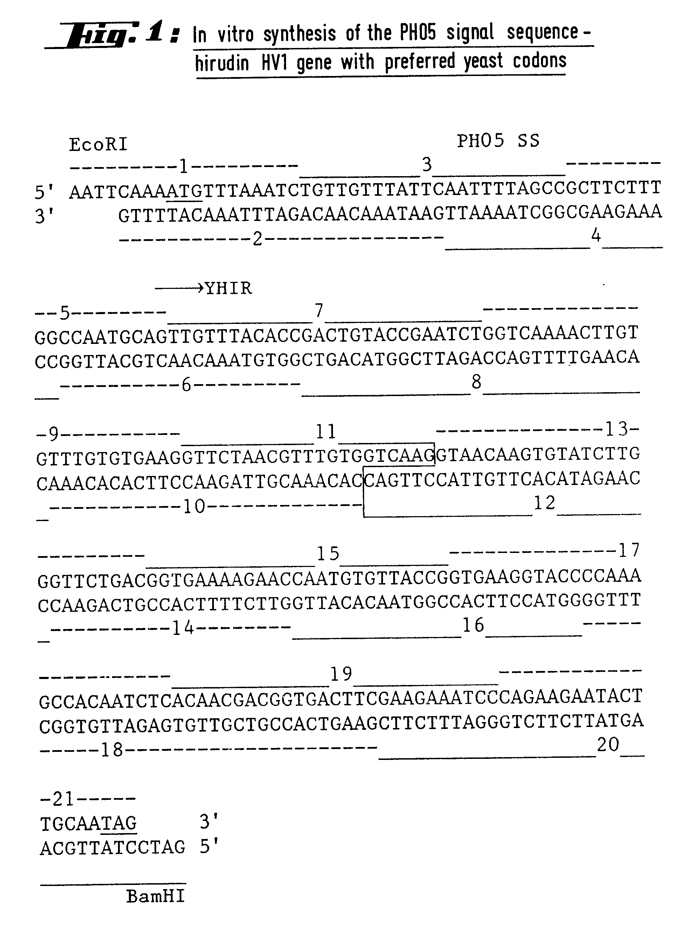

The coding sequence of the hirudin expression cassette is devised with preferred yeast codons [B. Hall J. Biol. Chem. <u>257</u> (1982) 3026] to guarantee optimal translation of the hirudin mRNA. The coding sequence contains the PH05 signal sequence fused in frame to the coding sequence of desulphatohirudin HV1. The 5' end of the synthetic DNA contains the sticky ends of the EcoRI restriction site. At the 3' end the stop codon TAG is immediately followed by the sticky ends of the BamHI site. The sequence of the 257 bp EcoRI-BamHI DNA fragment is shown in Fig. 1.

Fig. 1 also indicates the strategy for the <u>in vitro</u> synthesis of the double-stranded DNA fragment. 21 oligodeoxynucleotides are synthesized using the phosphor-amidite method [M.H. Caruthers, in: Chemical and Enzymatic Synthesis of Gene Fragments (H.G. Gassen and A. Lang, Eds.), Verlag Chemie, Weinheim, FRG] on an Applied Biosystems Model 380B synthesizer. The sequence of the individual oligonucleotides is shown in Fig. 1. The overlaps are unique. The lyophilized oligonucleotides are redissolved in 50 mM Tris-HCl pH 8.0 at a concentration of 10 pmoles/µl. The 21 oligonucleotides are allocated to two groups; [A] No. 1-11 representing the 5' halve of the DNA fragment, [B] No. 12-21 for the 3' halve. The 2 groups are treated separately. 10 pmoles each of the oligonucleotides of a group are mixed. The oligo's are phosphorylated in 20 µl of 25 mM Tris-HCl pH 8.0, 10 mM MgCl$_2$, 10 mM NaCl, 3 mM DTT, 0.4 mM ATP and 8 units of polynucleotide kinase (Boehringer) for 1 h at 37°C. After 30 min at room temperature both mixtures (A and B) are each heated for 5 min at 95°C in a waterbath. The samples are allowed to cool slowly to room temperature in the waterbath overnight. The annealed oligonucleotide mixtures A and B are then stored on ice.

Plasmid pBR322 is cut to completion with EcoRI and BamHI. The large, 4 kb fragment is isolated on a preparative 0.6 % agarose gel. The DNA is recovered by electroelution, purified by DE52 ion exchange chromatography and ethanol precipitation as described in Example 2. The DNA is redissolved in H₂O at a concentration of 0.4 pmoles/μl.

10 μl of the annealed oligonucleotide mixture A (5 pmoles each of oligos 1-11), 9.5 μl of mixture B (5 pmoles each of oligos 12-21), 0.4 pmoles of the 4 kb EcoRI-BamHI fragment of pBR322 and 400 units of T4 DNA ligase (Biolabs) are incubated for 16 h at 15°C.

10 μl aliquots are used to transform competent E. coli HB 101 Ca⁺⁺ cells. 12 transformed, ampicillin resistant colonies are grown individually in LB medium containing 100 μg/ml of ampicillin. Plasmid DNA is prepared by the method of Holmes et al. [Anal. Biochem. 114 (1981) 193] and analysed by EcoRI and BamHI restriction digests. Plasmid DNAS with the 257 bp EcoRI-BamHI insert are further analysed by DNA sequencing on both strands. Oligonucleotides 3, 11, 12, 14 and 20 (see Fig. 1) are used as sequencing primers. One clone with a correct sequence on both DNA strands is selected and referred to as pBR322/YHIR.

Example 2: Construction of plasmid pJDB207/GAPFL-YHIR

pJDB207/GAPFL-YHIR is a yeast plasmid for the expression of desulphatohirudin variant HV1 under the control of a short, constitutive promoter of the yeast glyceraldehyd-3-phosphate dehydrogenase (GAPDH) gene. The coding sequence of desulphatohirudin consists of preferred yeast codons.

10 μg of plasmid pBR322/YHIR are digested with restriction endonucleases BamHI and EcoRI. The 257 bp EcoRI-BamHI fragment is separated from other DNA fragments on a 1.2 % preparative agarose gel. The DNA bands are stained by ethidiumbromide and visualized under UV light at 360 nm. The 257 bp DNA band is cut from the gel and electroeluted in 0.2 x TBE buffer (TBE: 90 mM Tris-base, 90 mM boric acid, 2.5 mM EDTA, pH 8.3) for 45 min at 100 mA. After changing polarity for 45 sec, the DNA solution is collected and adjusted to 0.15 M NaCl. The DNA is adsorbed to a 100 μl bed of DE52 ion exchanger (Whatman) and eluted in 400 μl of high salt buffer (10 mM Tris-HCl pH 8.0, 1 mM EDTA, 1.5 M NaCl). The DNA is ethanol precipitated and resuspended in H₂O at a concentration of 0.1 pmoles/μl.

Plasmid pJDB207/GAPFL-HIR (European Patent Application No. 225 633) contains the synthetic gene for desulphatohirudin (based on the E. coli codon usage) fused in frame to the signal sequence of yeast acid phosphatase (PHO5). The gene is expressed under the control of a short constitutive glyceraldehyd-3-phosphate dehydrogenase (GAPFL) promoter of yeast on shuttle vector pJDB207. 10 μg of plasmid pJDB207/GAPFL-HIR are digested with SalI and EcoRI. The 478 bp SalI-EcoRI fragment contains the Sal-Bam pBR322 part and the GAPFL promoter. The DNA fragment is isolated on a 0.8 % preparative agarose gel, electroeluted and purified by DE52 chromatography and ethanol precipitation. The DNA is resuspended in H₂O at a concentration of 0.1 pmoles/μl. 5 μg of pJDB207/GAPFL-HIR are digested with SalI and BamH. The large 6.7 kb vector fragment is isolated as described above.

0.2 pmoles of the 478 bp SalI-EcoRI promoter fragment, 0.2 pmoles of the 257 bp EcoRI-BamHI fragment containing the PHO5 signal sequence and the synthetic hirudin gene (yeast codons) and 0.1 pmoles of the 6.7 kb vector fragment are ligated in 10 μl of 60 mM Tris-HCl pH 7.5, 10 mM MgCl₂, 5 mM DTT, 1 mM ATP and 200 units of T4 DNA ligase (Biolabs) for 6 h at 15°C. A one μl aliquot of the ligation mixture is used to transform competent E. coli HB 101 cells.

12 transformed, ampicillin resistant colonies are grown individually in LB medium containing 100 μg/ml of ampicillin. Plasmid DNA is prepared by the method of Holmes et al. (supra) and analysed by SalI/HindIII double digests. A single clone with the expected restriction pattern is referred to as pJDB207/GAPFL-YHIR.

In an analogous manner the construction can be performed with a 543 bp SalI-EcoRI promoter fragment of plasmid pJDB207/GAPEL-HIR (European Patent Application No. 225 633). The resulting new plasmid is referred to as pJDB207/GAPEL-YHIR.

Example 3: Construction of plasmid pJDB207/PHO5(-173)-HIR

pJDB207/PHO5(-173)-HIR is a yeast plasmid for the expression of desulphatohirudin variant HV1 under the control of a short PHO5 promoter. The PHO5(-173) promoter element comprises the nucleotide sequence of the yeast PHO5 promoter from position -9 to -173 (BstEII restriction site), but has no upstream regulatory sequences (UAS). The PHO5(-173) promoter therefore behaves like a constitutive promoter.

Plasmid pJDB207/PHO5(Eco)-HIR (EP 225 633) contains the full length, regulated PHO5 promoter with an EcoRI site introduced at position -8 with respect to the ATG of the PHO5 signal sequence and the coding sequence of desulphatohirudin which is followed by the PHO5 transcription termination fragment. This example describes the replacement of the regulated PHO5 promoter by the short PHO5(-173) promoter element.

20 μg of plasmid pJDB207/PHO5(Eco)-HIR are digested with BstEII. The sticky ends of the restriction fragments are filled in a reaction with Klenow DNA polymerase (1 unit/μg DNA) in 200 μl of 60 mM Tris-HCl pH 7.5, 10 mM MgCl₂, 0.1 mM each of dATP, dCTP, dGTP, TTP for 30 min at room temperature. After phenol extraction the DNA is ethanol precipitated.

4.16 μg of BamHI linker (5'-CGGATCCG-3', Biolabs) are phosphorylated in 100 μl of 60 mM Tris-HCl pH 7.5,

10 mM MgCl2, 5 mM DTT, 0.5 mM ATP and 18 units of T4 polynucleotide kinase (Boehringer) for 45 min at 37°C. After 10 min at 75°C the reaction mixture is slowly cooled to room temperature. The annealed oligonucleotide linkers are stored at -20°C.

4 pmoles of the [BstEll]/blunt end fragments of plasmid pJDB207/PH05(Eco)-HIR are incubated for 16 h at 15°C with a 100fold excess of the phosphorylated and annealed BamHI linker in 208 µl of 60 mM Tris-HCl pH 7.5, 10 mM MgCl2, 5 mM DTT, 3.5 mM ATP and 800 units of T4 DNA ligase (Biolabs). After inactivation of the ligase for 10 min at 85°C the excess linkers are removed by precipitation of the DNA in the presence of 10 mM EDTA, 300 mM sodium acetate pH 6.0 and 0.54 volumes of isopropanol. The DNA is digested with BamHI and EcoRI. The DNA fragments are separated on a 0.8 % preparative agarose gel. The 172 bp BamHI-EcoRI promoter fragment is recovered from the gel by electroelution and ethanol precipitation. The DNA is resuspended at a concentration of 0.1 pmoles/µl.

Plasmid pJDB207/PH05(Eco)-HIR is digested with EcoRI and HindIII. The 643 bp EcoRI-HindIII fragment is isolated as described above. The DNA fragment contains the PH05 signal sequence fused in frame to the coding sequence of desulphatohirudin and the PH05 transcription termination fragment. The plasmid is also cut with HindIII and BamHI. The 6.6 kb vector fragment is isolated.

0.2 pmoles each of the 172 bp BamHI-EcoRI fragment and the 643 bp EcoRI-HindIII fragment and 0.1 pmoles of the 6.6 kb vector fragment are ligated in 10 µl of 60 mM Tris-HCl pH 7.5, 10 mM MgCl2, 5 mM DTT, 1 mM ATP and 400 units of T4 DNA ligase (Biolabs) for 6 h at 15°C. A one µl aliquot of the ligation mixture is added to 100 µl of calcium-treated, transformation-competent E. coli HB101 cells.

12 transformed, ampicillin resistant colonies are grown in LB medium containing 100 µg/ml of ampicillin. Plasmid DNA is prepared and analysed by BamHI and SalI/HindIII digests. One clone with the expected restriction fragments is selected and referred to as pJDB207/PH05(-173)-HIR.

Example 4: Construction of plasmid pDP34

Yeast 2 micron covalently closed circle DNA is isolated from Saccharomyces cerevisiae strain S288C. Cells are incubated with 5 µg/ml of Zymolyase (100,000 units/µg) for 20 min at 37°C to digest the cell walls. The spheroplasts are lysed with 2 % SDS. EDTA is then added to 25 mM, ethidium bromide to 1 mg/ml and caesium chloride to a final density of 1.55 g/ml. Plasmid DNA is separated from the chromosomal DNA by ultracentrifugation for 42 hours at 42,000 rpm at 15°C. The 2 micron plasmid DNA is cut from the gradient with a syringe. The ethidium bromide is removed by extraction with NaCl-saturated isopropanol and the plasmid DNA is finally ethanol precipitated. The purified two-micron plasmid DNA is then linearised with PstI and cloned into the PstI site of pUC19 [J. Norrander et al., Gene 26 (1983), 101] to give plasmid pDP31.

Plasmid pJDB207 is digested with the restriction enzymes KpnI and HpaI. The resulting 0.55 kb HpaI-KpnI fragment contains the junction between the 2 micron sequence and the defective promoter of the dLEU2 gene.

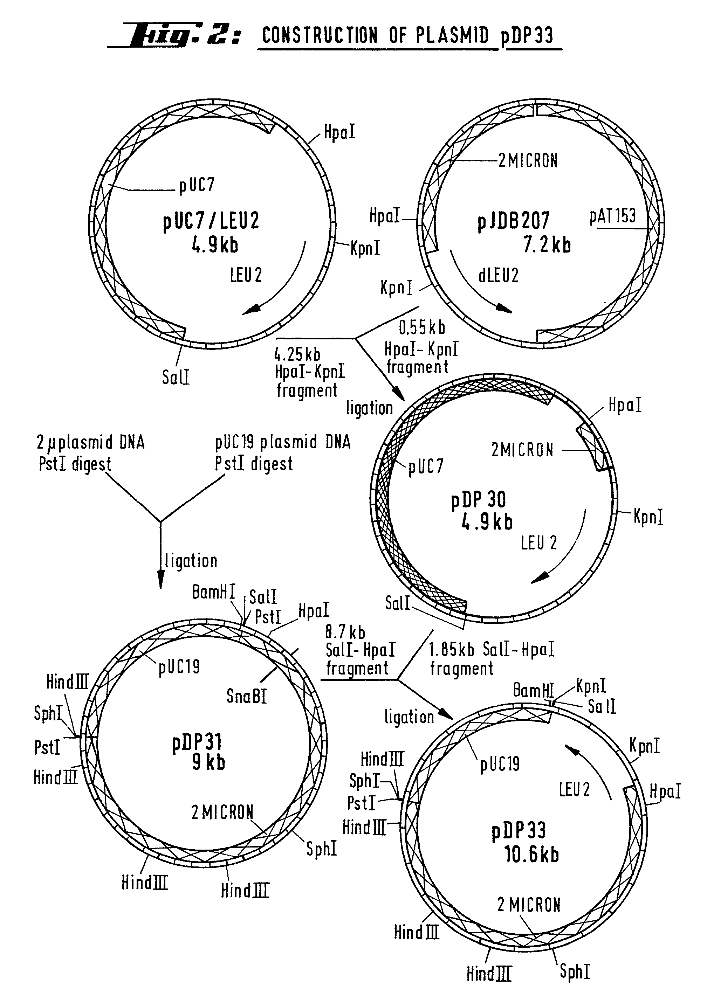

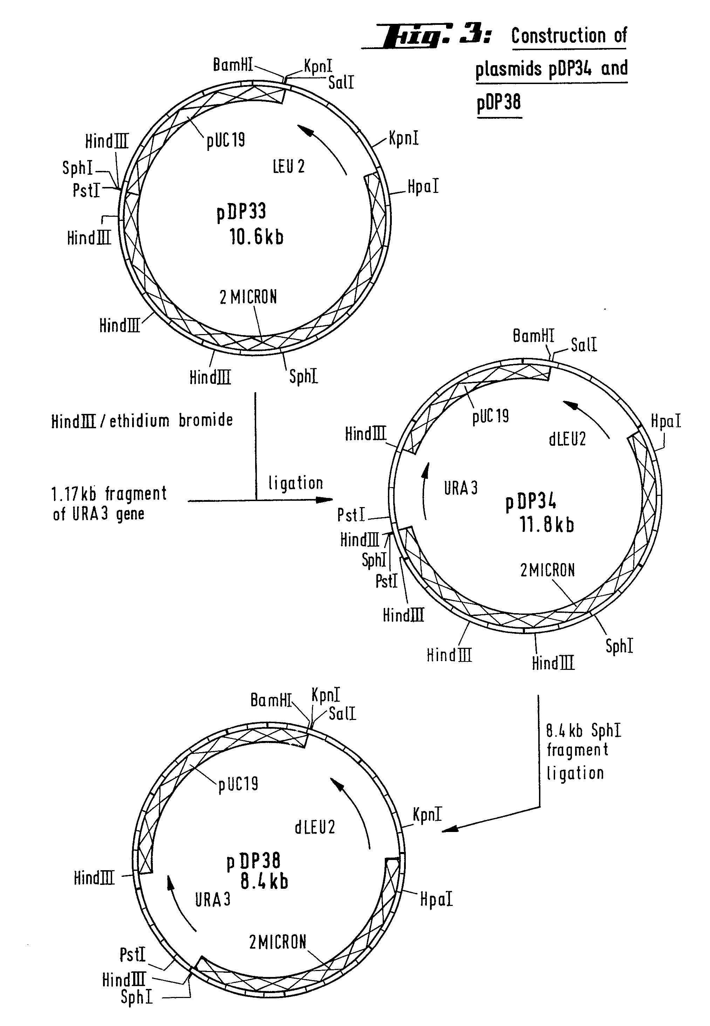

Plasmid pUC7/LEU2 contains the yeast genomic 2.2 kb XhoI-SalI fragment of the LEU2 gene [A. Andreadis et al., Cell 31 (1982), 319] cloned into the SalI site of the plasmid pUC7 [J. Vieira et al., Gene 19 (1982), 259]. Plasmid pUC7/LEU2 is cut with KpnI and HpaI. The 4.25 kb KpnI-HpaI fragment is ligated to the 0.55 kb HpaI-KpnI fragment of pJDB207. This results in plasmid pDP30 where the original two micron/dLEU2 fusion as in plasmid pJDB207 is placed in front of the LEU2 gene with its complete terminator. pDP30 is digested with HpaI and SalI and the 1.85 kb fragment containing the complete LEU2 gene is purified and cloned into the 8.7 kb SalI-HpaI fragment of plasmid pDP31. The resulting plasmid, pDP33 (see Fig. 2), is linearised by partial digestion with HindIII in the presence of 50 µg/ml ethidium bromide [M. Oesterlund et al., Gene 20 (1982) 121] and ligated with the 1.17 kb HindIII fragment containing the URA3 gene [M. Rose et al., Gene 29 (1984), 113]. Insertion of the URA3 gene is selected for by transformation into the E. coli strain pyrF [M. Rose et al., supra]. A positive clone is referred to as plasmid pDP34 (see Fig. 3).

pDP34 is a yeast-E. coli shuttle vector with the ampicillin resistance marker for E. coli and the URA3 and dLEU2 yeast selective markers. It contains the complete 2 micron sequence in the A form and is REP1, REP2 and FLP proficient.

Example 5: Cloning of hirudin expression cassettes into pDP34

Plasmid pDP34 is digested with BamHI. The sticky ends of the restriction site are filled in a reaction with Klenow DNA polymerase (T. Maniatis et al., in: "Molecular Cloning. A Laboratory Manual", Cold Spring Harbor Laboratory, 1982). The DNA is further cut with SalI and the 11.8 kb vector fragment is isolated on a preparative 0.6 % agarose gel. The DNA is recovered by electroelution and ethanol precipitation. Different expression cassettes are cloned into the pDP34 vector fragment between the SalI and [BamHI]/blunt end sites.

Plasmid pJDB207/GAPFL-YHIR is digested with HindIII. The sticky ends are converted to blunt ends by Klenow DNA polymerase. The DNA is ethanol precipitated and further digested with SalI. The 1.1 kb SalI-[HindIII]/blunt end fragment contains the complete expression cassette with pBR322 sequences, the GAPFL promoter, the PH05 signal sequence fused in frame to the coding sequence (preferred yeast codons) of desulphatohirudin and the PH05 transcription termination fragment. The 1.1 kb fragment is isolated on a preparative 0.8 % agarose gel, recovered from the gel by electroelution and purified by DE52 ion exchange chromatography and ethanol precipitation. 0.2 pmoles of the 1.1 kb fragment and 0.1 pmoles of the 11.8 kb vector fragment are ligated in 10 µl of 60 mM Tris-HCl pH 7.5, 10 mM MgCl2, 5 mM DTT, 3.5 mM ATP and 400 units of T4 DNA ligase (Biolabs) for 16 h at 15°C. A one µl aliquot is used to transform E. coli HB101 Ca$^{2+}$

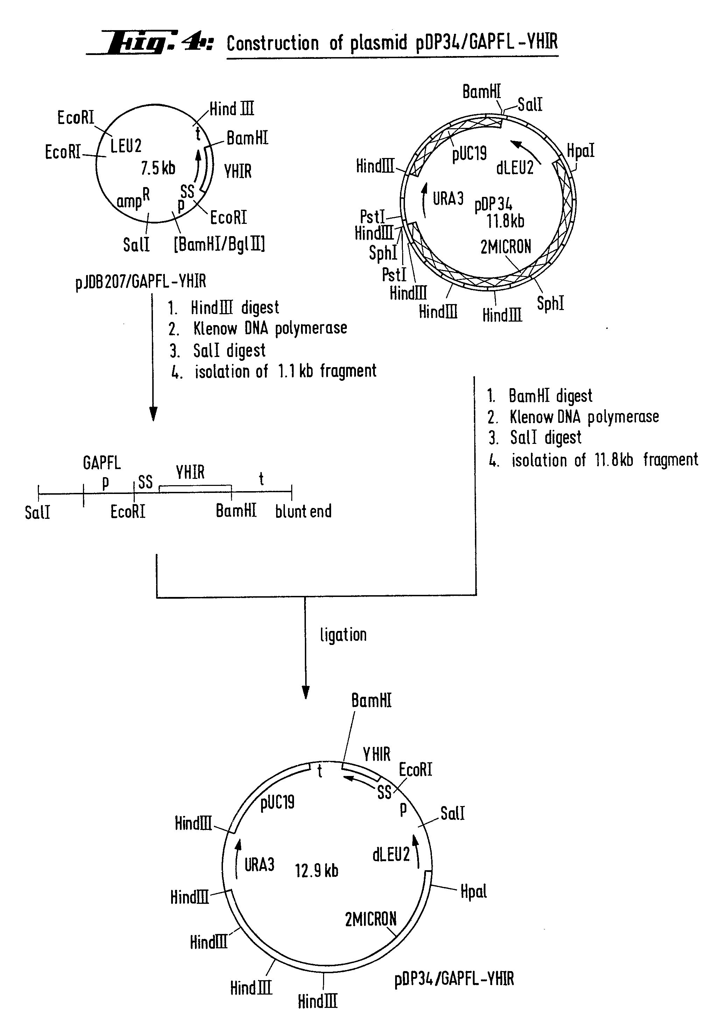

cells. 5 transformed, ampicillin resistant colonies are analysed. Plasmid DNA is digested with BamHI and Sall/BamHI. One clone with the correct restriction fragments is selected and referred to as pDP34/GAPFL-YHIR (see Fig. 4).

In an analogous manner the 1.2 kb Sall-[HindIII]/blunt end fragment of pJDB207/GAPEL-YHIR (see Example 2) is cloned into the pDP34 vector, which results in plasmid pDP34/GAPEL-YHIR.

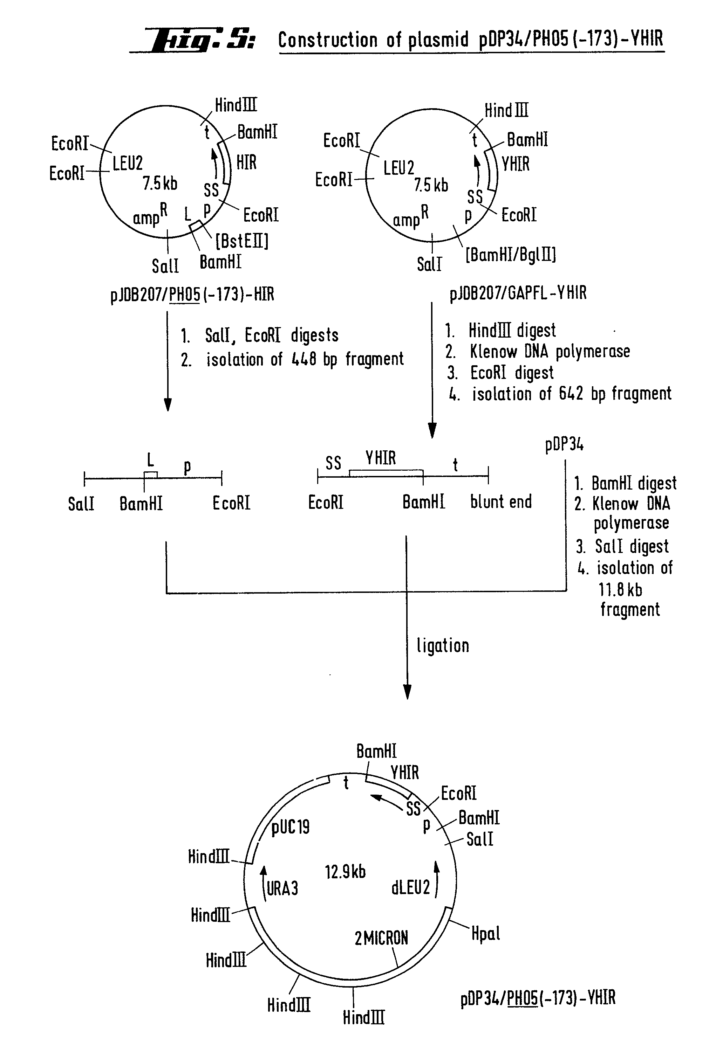

Plasmid pJDB207/PH05(-173)-HIR is digested with Sall and EcoRI. The 448 bp Sall-EcoRI fragment is isolated as described. The DNA fragment contains the Sall-BamHI part of pBR322 and the short constitutive PH05(-173)-promoter (see Example 3). Plasmid pJDB207/GAPFL-YHIR is digested with HindIII. The sticky ends are converted to blunt ends by Klenow DNA polymerase. The DNA is further digested with EcoRI. The 642 bp EcoRI-[HindIII]/blunt end fragment is isolated. It contains the PH05 signal sequence, the coding sequence of desulphatohirudin (with preferred yeast codons) and the PH05 transcription termination fragment. 0.2 pmoles each of the 448 bp Sall-EcoRI fragment and the 642 bp EcoRI-blunt end fragment and 0.1 pmoles of the 11.8 kb Sall-[BamHI]/blunt end vector fragment are ligated. Aliquots of the ligation mixture are used to transform E. coli HB 101 Ca$^{++}$ cells. Plasmid DNA of 12 transformants is analysed by BamHI and Sall/BamHI digests. One clone with the correct plasmid is selected and referred to as pDP34/PH05(-173)-YHIR (see Fig. 5).

### Example 6: Expression plasmids for the hirudin gene with E. coli codons

Expression plasmids containing the synthetic gene for desulphatohirudin variant HV1 based on the E. coli codon usage are constructed in a way analogous to the description in Example 5. The 1.1 kb Sall-[HindIII]/blunt end fragment of pJDB207/GAPFL-HIR (European Patent Application No. 225 633) is isolated and cloned into vector pDP34. The resulting expression plasmid is pDP34/GAPFL-HIR comprising the synthetic gene for desulphatohirudin based on preferred E. coli codons expressed under the control of the constitutive GAPFL promoter.

For a similar construction the 1.1 kb Sall-[HindIII]/blunt end fragment of pJDB207/PHO5(-173)-HIR (see Example 3) is cloned into pDP34. The resulting plasmid pDP34/PHO5(-173)-HIR contains the synthetic gene for desulphatohirudin (E. coli codons) under the control of the short constitutive PHO5(-173) promoter.

### Example 7: The hirudin expression cassette in plasmid pDP92

Vectors containing the complete two-micron sequence do not necessarily express all the functions of the genuine yeast two-micron circle. Open reading frames can be destroyed by cloning. Since no function had been known so far for the gene product of the "D" reading frame the unique PstI site withing this gene was used for cloning the dLEU2 gene [Beggs, J.D., Nature 275 (1978) 104-109] or inserting the pUC19 vector part as in plasmid pDP31 (see Example 4). Only recently it was suggested that the D gene product regulates expression of the FLP gene product [J.A.H. Murray et al., EMBO J. 6, 4205 (1987)]. To take full advantage of the two-micron circle a vector is constructed which is proficient for all the known two-micron functions including the D gene product.

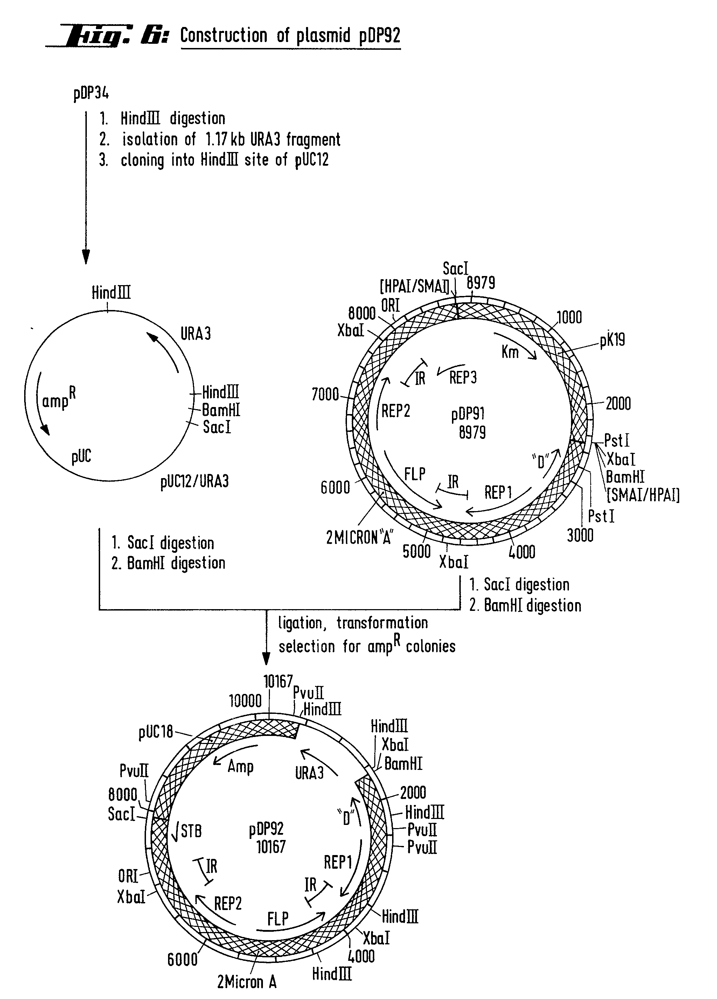

#### a) Construction of plasmid pDP92 (see Fig. 6)

Plasmid pDP31 (Example 4) is digested with PstI and HpaI resulting in three fragments. Plasmid pK19 [conferring kanamycin resistance; Pridmore, R.D., Gene 56 (1987) 309-312] is linearized with SmaI. The DNA fragments of both digests are phenol extracted and precipitated with ethanol. The DNA fragments are mixed and ligated. The ligation mixture is transformed [Hanahan, D. J., Mol. Biol. 166 (1983) 557-580] into competent E. coli JM109 cells [Yanisch-Perron, C. et al., Gene 33 (1985) 103-119], expressed for 2h at 37°C in LB medium and then plated on LB agar plates supplemented with 50 µg/ml of kanamycin, 30 µg/ml of XGal and 7 µg/ml of IPTG.

12 white, kanamycin-resistant colonies are grown. Plasmid DNA is analysed by XbaI and BamHI/KpnI digests. A single clone which has lost the pUC19 vector part of pDP31, restored the two-micron D reading frame by religation of the PstI site and which has the pK19 plasmid blunt end inserted into the HpaI site is referred to as pDP91. The plasmid contains the large HpaI-PstI and the small PstI-HpaI fragments of two-micron plasmid cloned into the SmaI site of pK19. By religation of the PstI sites the D reading frame is reconstituted.

The URA3 gene is isolated on a 1.17 kb HindIII fragment from plasmid pDP34 (see Example 4) and cloned into the unique HindIII site of plasmid pUC12. A clone with the URA3 gene inserted in the same orientation as the ampicillin resistance gene is referred to as pUC12/URA3. Plasmid pDP91 and pUC12/URA3 are both digested with SacI and BamHI resulting in two fragments each. The DNA fragments are mix-ligated and used to transform competent E. coli JM109 cells. Cells are plated on to LB agar plates supplemented with 100 µg/ml of ampicillin, 30 µg/ml of XGal and 7 µg/ml of IPTG.

12 white, ampicillin-resistant colonies are grown. Plasmid DNA is analysed by HindIII and PvuII digests. A single clone is referred to as pDP92, comprising the complete two-micron sequence proficient for all its known functions and the URA3 gene cloned into the pUC vector.

#### b) Cloning of hirudin expression cassettes into pDP92

In analogy to Example 5 pDP92 is digested with BamHI. The sticky ends are filled in a reaction with Klenow DNA polymerase. The DNA is further cut with Sall. The 10.2 kb vector fragment is isolated. The 1.1 kb SalI-[HindIII]/blunt end fragment of plasmid pJDB207/GAPFL-YHIR is isolated and ligated to the vector fragment.

6 transformed, ampicillin-resistant colonies are analysed. Plasmid DNA is digested with BamHI, PstI and SalI/BamHI. One clone with the expected restriction fragments is selected and referred to as pDP92/GAPFL-YHIR. In a similar way plasmid pDP92/GAPEL-YHIR is obtained using the 1.2 kb SalI-[HindIII]/blunt end fragment of pJDB207/GAPEL-YHIR (see Example 2).

Plasmid pDP92/PHO5(-173)-YHIR is constructed as described in Example 5 using the 10.2 kb SalI-[BamHI]/blunt end pDP92 vector fragment (see above).

Example 8: Construction of two-micron DNA free Saccharomyces cerevisiae host strains

In order to remove the endogenous two-micron plasmid, in a first step a deletion is introduced in the URA3 gene of strain HT246 (DSM 4084; α, leu 2-3, leu 2-112, prb) to make the strain auxotrophic for uracil. HT246 is transformed with 1 μg of plasmid YEp13 [Broach, J.R., Strathern, J.N., Hicks, J.B. (1979) Gene 8, 121-123] using the transformation protocol described by Hinnen et al. [Proc. Natl. Acad. Sci. USA 75, 1929 (1978)]. 10 μg of plasmid pUC12ura3Δ containing a deletion in the URA3 gene [Sengstag, Ch., Hinnen, A., Nucleic Acids Research 15, 233-246 (1987)] are added along with plasmid YEp13. Roughly 3000 leucine prototrophic transformants are resuspended in 5 ml minimal medium (Difco Yeast Nitrogen Base without amino acids to which 2 % glucose, 0.1 % leucine, 0.1 % uracil and 0.25 % fluoroorotic acid are added) in a small shake flask and incubated for 60 hours at 30°C and 180 r.p.m. Transformants which grow are resistant to the toxic analogue fluoroorotic acid and carry therefore a replacement in the chromosomal URA3 gene by ura3Δ. The grown cells are plated out on full medium composed of (g/l): Peptone 20, yeast extract 10, glucose 20, and after growth for 48 h at 30°C replica-plated onto minimal medium (Difco) yeast nitrogen base without amino acids. Supplemented with 2 % glucose and 0.1 % leucine) to detect uracil auxotrophs. Several auxotrophs are picked and tested for plasmid YEp13 loss conferring leucine auxotrophy. One individual colony (designated Tr889) requiring leucine and uracil is picked and used for further experimentation.

Tr889 is transformed with plasmid pDP38 [obtained from plasmid pDP34 by digestion with SphI and religation of the resulting 8.4 kb fragment; see Fig. 3], which carries both marker genes LEU2 and URA3 (transformation protocol, supra). Transformed yeast cells are first selected on yeast minimal media plates deficient in uracil, supplemented with leucine and then replica-plated onto minimal medium deficient in leucine and supplemented with uracil. 10 weakly growing colonies are picked and individually grown in liquid full medium (supra) over about a hundred generations. By doing so, the cells lose the pDP38 plasmid and - to a certain percentage - simultaneously also the endogenous two-micron plasmid. 10 uracil and leucine requiring colonies are picked, DNA is prepared, the DNA is digested to completion with PstI and probed with 32P-labelled yeast two-micron DNA on Southern blots. One isolate without any hybridisation signal is referred to as H449 (a, leu2-3, leu2-112, ura3Δ, prb, cir°), an isogenic two-micron free (cir°) derivative of yeast strain HT246.

Example 9: The hirudin expression cassette in plasmid pDP96

pDP96 has been constructed as an alternative to pDP92. The essential difference in the construction of pDP96 is the use of the unique SnaBI site in the two-micron circle for cloning as compared to the HpaI site in pDP92. In pDP96 all known open reading frames of the two-micron circle, including the D reading frame, are intact. Therefore, the vector should be proficient for all known two-micron functions.

a) Construction of plasmid pDP96

Plasmid pDP31 (Example 4) is digested with PstI and SnaBI resulting in three fragments. Plasmid pK19 [conferring kanamycin resistance; Pridmore, R.D., Gene 56 (1987) 309-312] is linearized with SmaI. The DNA fragments of both digests are phenol extracted and precipitated with ethanol. The DNA fragments are mixed and ligated. The ligation mixture is transformed [Hanahan, D. J., Mol. Biol. 166 (1983) 557-580] into competent E. coli JM109 cells [Yanisch-Perron, C. et al., Gene 33 (1985) 103-119], expressed for 2h at 37°C in LB medium and then plated on LB agar plates supplemented with 50 μg/ml of kanamycin, 30 μg/ml of XGal and 7 μg/ml of IPTG.

12 white, kanamycin-resistant colonies are grown. Plasmid DNA is analysed by XbaI and BamHI/KpnI digests. A single clone which has lost the pUC19 vector part of pDP31, restored the two-micron D reading frame by religation of the PstI site and which has the pK19 plasmid blunt end inserted into the SnaBI site is referred to as pDP95. The plasmid contains the large SnaBI-PstI and the small PstI-SnaBI fragments of the two-micron plasmid cloned into the SmaI site of pK19. By religation of the PstI sites the D reading frame is reconstituted.

Plasmid pUC18/URA3 consists of the yeast 1.17 kb URA3 gene (HindIII fragment) cloned at the HindIII site of the E. coli vector pUC18 with the URA3 gene inserted in the opposite orientation as the ampicillin resistance gene. pUC18/URA3 is partially digested with the restriction enzyme HindIII in the presence of 50 μg/ml ethidium bromide for 1h at 37°C. The addition of ethidium bromide to the digestion allows a first site to be digested by HindIII, but the subsequent intercalation of ethidium bromide into the linearised DNA interferes with the digestion of the second site, thus enriching for the linearised plasmid DNA. The restriction enzyme and ethidium bromide are removed by two consecutive phenol extractions and the DNA is ethanol precipitated. This DNA is then treated with the DNA polymerase large fragment (Klenow enzyme) to fill in the 5'

overhangs of the HindIII sites. This end repaired DNA is run on an agarose gel to separate the various fragments, including the enriched, end repaired linear. The 3.35 kb pUC18/URA3 linear DNA is cut out of the gel and electro-eluted. This DNA is then self ligated with T4 DNA ligase, transformed into competent E. coli JM109 cells and plated onto YT plates supplemented with 50 μg/ml ampicillin. Colonies are screened as above to identify plasmid 'D', where the HindIII site at the pUC linker-array side of the URA3 gene has been end repaired creating a new unique NheI restriction site. Plasmid 'D' is digested with the restriction enzyme HindIII to completion and the 5' overhangs are filled in a reaction with Klenow DNA polymerase. This DNA is then mixed with a large excess of NotI linkers (GCGGCCGC), ligated with T4 DNA ligase, transformed into competent JM109 cells and plated onto TY plates supplemented with 50 μg/ml ampicillin. Colonies are screened as above and plasmid 'E' is identified, where the HindIII site has been end repaired and a NotI linker added. Plasmid 'E' is digested with the restriction enzyme SacI, and the 3' overhangs are repaired with T4 DNA polymerase. The DNA is then mixed with a large excess of NotI linkers and ligated with T4 DNA ligase. This ligation mixture is transformed into competent E. coli JM109 cells and plated onto YT plates supplemented with 50 μg/ml ampicillin. Colonies are screened as above and plasmid pUC18/URA3-N is identified, where the pUC18 sequences are now flanked by NotI restriction sites (plasmids 'D' and 'E' are only intermediates in the construction of pUC18/URA3-N).

Plasmid pDP95 and pUC18/URA3-N are both digested with KpnI and BamHI resulting in two fragments each. The DNA fragments are mix-ligated and used to transform competent E. coli JM109 cells. Cells are plated on to LB agar plates supplemented with 100 μg/ml of ampicillin, 30 μg/ml of XGal and 7 μg/ml of IPTG.

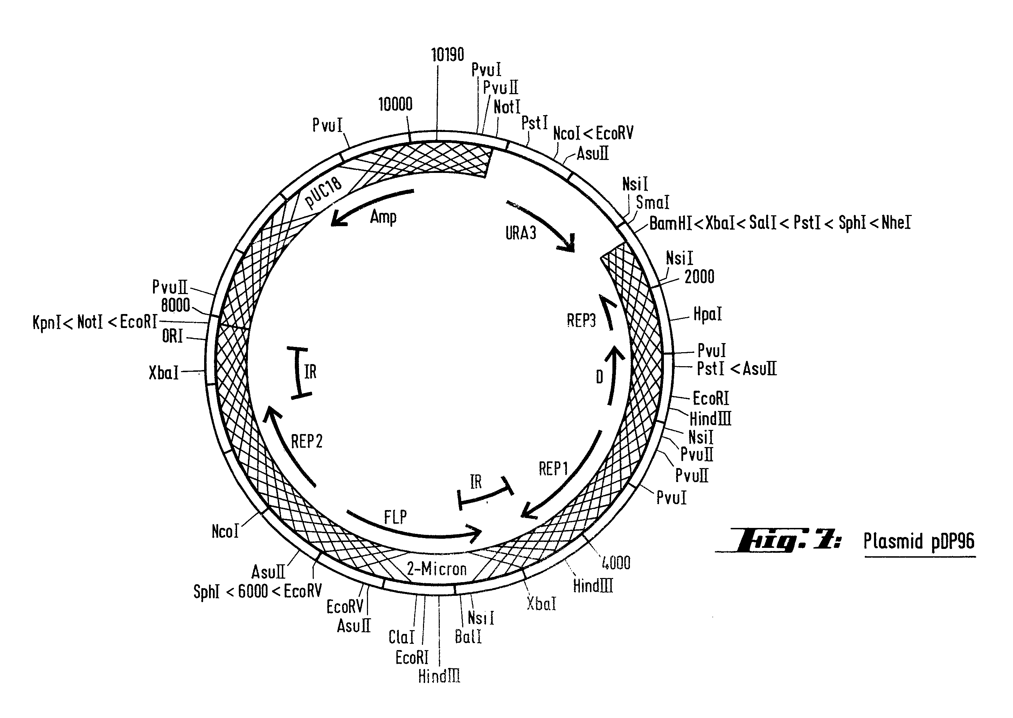

12 white, ampicillin-resistant colonies are grown. Plasmid DNA is analysed by HindIII and PvuII digests. A single clone is referred to as pDP96, comprising the complete two-micron sequence proficient for all its known functions and the URA3 gene cloned into the pUC vector (see Fig. 7).

b) Cloning of hirudin expression cassettes into pDP96

In analogy to Example 5 pDP96 is digested with BamHI. The sticky ends are filled in a reaction with Klenow DNA polymerase. The DNA is further cut with SalI. The 10.2 kb vector fragment is isolated. The 1.1 kb SalI-[HindIII]/blunt end fragment of plasmid pJDB207/GAPFL-YHIR is isolated and ligated to the vector fragment.

6 transformed, ampicillin-resistant colonies are analysed. Plasmid DNA is digested with BamHI, PstI and SalI/BamHI. One clone with the expected restriction fragments is selected and referred to as pDP96/GAPFL-YHIR. In a similar way plasmid pDP96/GAPEL-YHIR is obtained using the 1.2 kb SalI-[HindIII]/blunt end fragment of pJDB207/GAPEL-YHIR (see Example 2).

Plasmid pDP96/PHO5(-173)-YHIR is constructed as described in Example 5 using the 10.2 kb SalI-[BamHI]/blunt end pDP96 vector fragment (see above).

Example 10: Transformation of S. cerevisiae strain H449

Saccharomyces cerevisiae strain H449 is transformed with plasmids
pDP34/PHO5(-173)-HIR
pDP34/GAPFL-HIR
pDP34/GAPEL-HIR
pDP34/PHO5(-173)-YHIR
pDP34/GAPFL-YHIR
pDP34/GAPEL-YHIR
pDP92/PHO5(-173)-YHIR
pDP92/GAPFL-YHIR
pDP92/GAPEL-YHIR
pDP96/GAPFL-YHIR
pDP96/GAPEL-YHIR
pDP96/PHO5(-173)-YHIR
using the transformation protocol described by Hinnen et al. (supra). Transformed yeast cells are selected on yeast minimal medium plates supplemented with leucine and deficient in uracil. Single transformed yeast cells are isolated and referred to as
Saccharomyces cerevisiae H449/pDP34/PHO5(-173)-HIR
Saccharomyces cerevisiae H449/pDP34/GAPFL-HIR
Saccharomyces cerevisiae H449/pDP34/GAPEL-HIR
Saccharomyces cerevisiae H449/pDP34/PHO5(-173)-YHIR
Saccharomyces cerevisiae H449/pDP34/GAPFL-YHIR
Saccharomyces cerevisiae H449/pDP34/GAPEL-YHIR
Saccharomyces cerevisiae H449/pDP92/PHO5(-173)-YHIR
Saccharomyces cerevisiae H449/pDP92/GAPFL-YHIR
Saccharomyces cerevisiae H449/pDP92/GAPEL-YHIR
Saccharomyces cerevisiae H449/pDP96/GAPFL-YHIR
Saccharomyces cerevisiae H449/pDP96/GAPEL-YHIR
Saccharomyces cerevisiae H449/pDP96/PHO5(-173)-YHIR

Example 11: Fermentation of transformed yeast strains on a laboratory scale

Cells of Saccharomyces cerevisiae H449/pDP34/PHO5(-173)-YHIR and of Saccharomyces cerevisiae H449/pDP34/GAPFL-YHIR are each grown in two subsequent precultures of 10 ml minimal medium composed of (g/l):

| | |
|---|---|
| Difco Yeast Nitrogen Base | 6.7 |
| asparagine | 10 |
| leucine | 1 |
| glucose | 20 |

The first preculture is grown for 60 h at 28°C and 180 r.p.m. The second preculture is inoculated with 2 % of the first preculture and incubated for 24 h at 28°C and 180 r.p.m.

The main culture medium is composed of (g/l):

| | |
|---|---|
| yeast extract | 49 |
| glucose | 5 |
| fructose | 57 |
| $NH_4NO_3$ | 0.5 |
| $MgSO_4 \times 7\ H_2O$ | 1.0 |
| $CaCO_3$ | 5.0 |
| $Ca_3(PO_4)_2$ | 2.0 |

The main culture is inoculated with about $2 \times 10^6$ cells/ml and incubated up to 72 h at 28°C and 180 r.p.m. Approximately $1 \times 10^9$ cells/ml are obtained at the end of the fermentation. At several time points during the fermentation aliquots of the cultures are taken, the cells removed by centrifugation and the culture supernatant analysed for desulfatohirudin by HPLC (infra).

Example 12: Production of desulphatohirudin variant HV1 on a 50 l scale

A working cell bank of the 2 μ free strain Saccharomyces cerevisiae H449/pDP34/GAPFL-YHIR, has been used as a source of inoculum for the production of desulphatohirudin on a 50 l scale.

Ampoules of the working cell bank are preserved in the vapour phase in a liquid nitrogen container. The contents of one ampoule are used to inoculate a shake flask culture comprising a selective medium consisting of (g/l)

| | |
|---|---|
| yeast nitrogen base | 8.4 |
| L-asparagin monohydrate | 11.4 |
| L-histidin | 1.0 |
| L-leucine | 0.1 |
| D-glucose monohydrate | 20.0 |

The 500 ml flask contains 100 ml medium and is incubated for 48 h at 28°C on an orbital shaker at a shaking speed of 180 rev/min.

The second shake flask pre-culture comprises of the same medium of which 600 ml are contained in a 2 l flask which has four baffles. The inoculum level from the first pre-culture is 5 % (30 ml) and the flasks are incubated for 48 h at 28°C on an orbital shaker at a speed of 120 rev/min.

A third pre-culture is fermented in a 50 l stainless steel bioreactor equipped with 4 baffles and a single disk turbine agitator with a diameter of 115 mm. The above medium is also used for this culture, the starting volume being 30 l. A single 2 l flask containing 600 ml culture is used to inoculate the 50 l reactor (2 %). The fermentation lasts for about 42 h at a temperature of 28°C. The stirrer speed is 600 rev/min, aeration rate 1 vvm and the reactor is operated with an overpressure of 0.3 bar.

A similar 50 l bioreactor, additionally equipped for fed-batch processes, is used for the desulphatohirudin production stage. A medium consisting of (g/l)

| | |
|---|---|
| meat peptone (Merck) | 5.0 |
| yeast extract | 30.0 |
| ammonium sulphate | 6.0 |
| magnesium sulphate heptahydrate | 1.0 |
| sodium chloride | 0.1 |
| potassium dihydrogenphosphate | 1.0 |
| D-glucose monohydrate | 10.0 |

is used for this stage (30 l). The inoculum level from the third preculture stage is optionally 2 %. The fermentation lasts for 48 h at a temperature of 28°C and the stirrer speed is set at 750 rev/min. The overpressure is initially set at 0.3 bar, but this can be raised to 1.0 bar during the course of the fermentation to maintain the dissolved oxygen tension above 20 % saturation. The initial air flow is 0.25 vvm but this is increased to 1 vvm after nine hours in order to ensure an adequate oxygen supply. The pH value falls during the early part of the fermentation to a value of 5.0 at which it is maintained by an automatic feed of ammonium hydroxide.

In simple batch culture the biomass level attained, and consequently the desulphatohirudin titre, is dependent upon the amount of the carbon source which is batched into the fermenter at the beginning. This in turn is dictated by the oxygen transfer capacity of the bioreactor and the need to avoid excessive production of ethanol by the growing yeast. These limitations can be overcome by using fed-batch technology. Thus rather little glucose is included in the start medium but a feed of glucose is made to support a considerably higher final biomass concentration and a desulphatohirudin titre approximately three times that reached in batch culture. In practice the feed is increased at intervals in a stepwise manner to a final feed rate of 175 g/h of glucose monohydrate.

Small additions of a silicone based antifoam are used to control foaming when necessary. A portion of the exit gas from the fermenter is analysed to provide information about the oxygen uptake and carbon dioxide evolution rate. The dissolved oxygen tension is measured on-line using a sterilizable electrode.

Samples are withdrawn at 6 hourly intervals throughout the process to allow monitoring of the glucose and ethanol concentrations, the desulphatohirudin titre by bio-assay and HPLC, and also to check the sterility. At the end of the fermentation process desulphatohirudin can be recovered from the culture supernatant.

Example 13: Analysis of hirudin compounds from the fermentation of S. cerevisiae strain H449/pDP34/GAPFL-YHIR

The supernatant of the fermentation broth (see Examples 11 and 12) is routinely subjected to HPLC analysis (experimental conditions No. 1, see below). Purified products are analyzed by additional methods, e.g. ion exchange chromatography (IEC) on Mono-Q column (experimental conditions No. 2) and high performance gel filtration chromatography (HPGFC), experimental conditions No. 3).

Experimental conditions No. 1:

Method

Reversed phase high performance liquid chromatography (RP-HPLC) with gradient elution
Column Type: Nucleosil 100-5 $C_{18}$ (Macherey-Nagel, Düren, FRG)
Particle Size: 5 µm
Dimensions: 4.0 x 120 mm
Sample volume: 50 µl
Protein load/injection 2.5 - 10 µg (50 - 200 µg/ml)
Flow rate: 1.5 ml/min
Counterpressure $\simeq$ 120 - 150 bar
Detection: at 215 nm (attenuation: 6)

Eluent A:
water (Analyzed HPLC Reagent, BAKER Chemicals, Deventer, Holland) with 0.1 % (v/v) trifluoroacetic acid (TFA)

Eluent B:
acetonitrile (HPLC-grade, Fluka) with 0.07 % (v/v) trifluoroacetic acid (TFA)

| Elution conditions | t(min) | %B (v/v) |
|---|---|---|
| | 0 | 10 |
| | 1 | 19 |
| | 9 | 19 |
| | 17 | 29 |
| | 20 | 95 |
| | 24 | 10 |
| | 30/0 | 10 |

.

Experimental conditions No. 2:

Method:
Ion exchange chromatography (IEC) on a strong anion exchanger stationary phase (pH-gradient elution)
Column Type: Mono-Q (Pharmacia, Uppsala, Sweden)
Dimensions: 5.0 x 50.0 mm
Sample volume: 100 µl
Protein load/injection: 25 - 100 µg (250 - 1000 µg/ml)
Flow rate: 2.0 ml/min
Counterpressure: ≃ 30 - 35 bar
Detection at 280 nm (attn. 5)

Eluent A:
50 mM $HCOONH_4$, pH 4.5

Eluent B:
50 mM $HCOONH_4$, pH 3.5

| Elution conditions: | t(min) | %B (v/v) |
|---|---|---|
| | 0 | 20 |
| | 5 | 20 |
| | 15 | 75 |
| | 15.1 | 100 |
| | 17.5 | 100 |
| | 17.6 | 20 |
| | 20/0 | 20 |

Experimental conditions No. 3:

Method:
High performance gelfiltration chromatography (HPGFC)
Column Type: Superose 12 (Pharmacia, Uppsala, Sweden)
Dimensions: 10.0 x 300 nm
Sample volume: 50 µl
Protein load/injection: 25 - 10 µg (500 - 2000 µg/ml)
Flow rate: 0.5 ml/min
Counterpressure: ≃ 9 bar
Detection: at 280 nm (attn. 5)
Eluent: 0.1 M $NH_4COOCH_3$, pH 5.0

15

| Eluent conditions: | t(min) | %B (v/v) |
|---|---|---|
| | 0 | 0 |
| | 5 | 0 |
| | 35 | 100 |
| | 40 | 100 |
| | 40.1 | 0 |
| | 50/0 | 0 |

Peaks of recombinant desulphatohirudin [= peak 1], of desulphatohirudin(1-64) [= peak 2], of desulphatohirudin(1-63) [= peak 3], of desulphatohirudin variants B6a [= peak 4], B6b [= peak 5] and B7 [= peak 6], of authentic hirudin from the leech Hirudo medicinalis [= peak 7] and of authentic desulphatohirudin [= peak 8; obtained by reacting natural hirudin [peak 7] with the enzyme arylsulphatase] are collected and subjected to thrombin inhibition test (Chromozym TH as chromogenic substrate) and to one or more analytical methods (see experimental conditions above). The results are compiled in the following Table 1:

Table 1:

| Experimental conditions No. | Retention times (min) | | | | | | | |
|---|---|---|---|---|---|---|---|---|
| | Peak 1 | Peak 2 | Peak 3 | Peak 4 | Peak 5 | Peak 6 | Peak 7 | Peak 8 |
| 1 (RP-HPLC, gradient) | 17.37 | 17.7 | 12.5 | 17.6 | 17.6 | 17.4 | 16.3 | 17.4 |
| 2 (IEC, Mono-Q) | 13.2 | 15.3 | 15.7 | 10.2 | 10.8 | 16.4 | n.d. | n.d. |
| 3 (HPGFC, Superose) | 27.2 | 27.2 | 27.3 | 27.2 | 27.2 | 27.1 | 27.2 | 27.2 |
| Thrombin Inhibition (%) | ~ 100 | ~ 50 | ~ 15 | ~ 90 | ~ 90 | ~ 90 | ~ 105 | ~ 95 |

n.d.: not determined

Example 14: Characterisation of recombinant desulphatohirudin and other hirudin compounds from the fermentation of S. cerevisiae strain H449/pDP34/GAPFL-YHIR

Recombinant yeast desulphatohirudin [= peak 1] (see Example 13) from the fermentation of S. cerevisiae strain H449/pDP34/GAPFL-YHIR is characterised and compared to the hirudin derivatives desulphatohirudin(1-64) [= peak 2], desulphatohirudin(1-63) [= peak 3], desulphatohirudin-variants B6a [= peak 4], B6b [= peak 5] and B7 [= peak 6], authentic hirudin from the leech Hirudo medicinalis [= peak 7] and to authentic desulphatohirudin [= peak 8].

A) Molecular weight determination

a) Apparent molecular weight

The hirudin compounds are analysed by standard SDS-PAGE (10 µg protein, 10 x 15 cm gel-size, 15 % polymerisation) or on a commercial PhastSystem (Pharmacia, Uppsala, Sweden) according to the manufacturers instructions (1 µg protein, 20 % homogeneous SDS-PAGE). Prior to Coomassie blue staining fixation is done with 3 % (v/v) β-mercaptoethanol for 40 min.

Results

A single band for all compounds is observed, corresponding to an apparent molecular weight of about 7000 Daltons. The relative mobilities ($R_f$-values) are summarized in the following Table 2:

Table 2:

| Peak No. | Relative mobility ($R_f$-value) | |
|---|---|---|
| | SDS-PAGE | PhastSystem |
| 1 | 1.0 | 1.0 |
| 2 | 1.0 | not assayed |
| 3 | 1.0 | not assayed |
| 4 | 0.99 | 0.91 |
| 5 | 0.99 | 0.91 |
| 6 | 1.03 | 1.06 |
| 7 | 1.0 | not assayed |
| 8 | 1.0 | 1.0 |

b) Chemical molecular weight determination by FAB-MS

The hirudin compounds are subjected to fast atom bombardment positive ion mass spectrometry (FAB-MS). Instrument: ZAB-HF mass spectrometer from VG-Analytical Ltd., Manchester; matrix: thioglycerol; Xenon bombardment; ion energy 10 KeV; extern calibration: $Cs_{26}J_{25}$ (molecular weight: 6887.9) and $Cs_{28}H_{27}$ (7147.76).

Results

The results are summarized and compared to the calculated values in the following Table 3:

Table 3:

| Peak No. | Empirical formula | Molecular weight | |
|---|---|---|---|
| | | calculated | found |
| 1 | $H_{287}H_{440}N_{80}O_{110}S_6$ | 6963.518 | 6963.44 |
| 2 | $H_{282}H_{432}N_{78}O_{108}S_6$ | 6835.39 | 6832.9 |
| 3 | $H_{276}H_{421}N_{77}O_{107}S_6$ | 6722.23 | 6719.3 |
| 4 | not assayed | | |
| 5 | not assayed | | |
| 6 | not assayed | | 6941 |

B) Determination of the amino acid composition

0.1 - 2.0 µg of pure hirudin compounds are hydrolysed for 24 h with 6N HCl at 110°C and then analysed (DABS-Cl method) as described by Chang et al. [Methods Enzymol. 91 (1983) 41-48; J. Chromatogr. 295 (1984) 193-200].

Results
The results summarized in Table 4 are compared to the calculated values and are as expected:

Table 4

| Amino acid | Peak 1 | | Peak 2 | | Peak 3 | |
|---|---|---|---|---|---|---|
| | calc. | found | calc. | found | calc. | found |
| ALA | 0 | 0 | 0 | 0 | 0 | 0 |
| ARG | 0 | 0 | 0 | 0 | 0 | 0 |
| ASN | 9 | 7.2 | 9 | 10.0 | 9 | 9.2 |
| CYS | 6 | 6.0 | 6 | 4.8 | 6 | 5.0 |
| GLN | 13 | 13.1 | 12 | 12.5 | 12 | 12.4 |
| GLY | 9 | 8.5 | 9 | 8.7 | 9 | 9.0 |
| HIS | 3 | 0.9 | 3 | 1.0 | 3 | 1.0 |
| ILE | 2 | 2.3 | 2 | 1.9 | 2 | 2.1 |
| LEU | 4 | 4.7 | 4 | 3.3 | 3 | 3.3 |
| LYS | 3 | 3.3 | 3 | 2.8 | 3 | 3.1 |
| MET | 0 | 0 | 0 | 0 | 0 | 0 |
| PHE | 1 | 1.1 | 1 | 1.1 | 1 | ·1.2 |
| PRO | 3 | 2.8 | 3 | 3.1 | 3 | 3.1 |
| SER | 4 | 4.0 | 4 | 4.1 | 4 | 4.0 |
| THR | 4 | 3.9 | 4 | 3.9 | 4 | 4.1 |
| TRP | 0 | n.d. | 0 | n.d. | 0 | n.d. |
| TYR | 2 | 1.9 | 2 | 1.8 | 2 | 1.9 |
| VAL | 4 | 3.7 | 4 | 3.1 | 4 | 3.4 |
| Total AA | 65 | | 64 | | 63 | |

C) Sequence analysis
For N-terminal amino acid sequence analysis 18 µg (2-3 nMol) of the pure hirudin compounds (or of tryptic fragments) are subjected to a conventional sequence analysis according to Edman and Begg [Europ. J. Biochem. 80 (1967)] on a gas-phase protein-sequencer (Mod. 470A, Applied Biosystems) and the N-terminal phenylthiohydantoin (PTH)-amino acids are determined by means of RP-HPLC. For C-terminal analysis the hirudin compounds are digested with carboxypeptidase Y and the released amino acids are determined in the amino acid analyser (DABS-C1 method, see above).

Results

```
Cycle                                    5                            10
expected     Val – Val – Tyr – Thr – Asp – Cys – Thr – Glu – Ser – Gly
found peak 1 Val – Val – Tyr – Thr – Asp –(Cys)– Thr – Glu – Ser – Gly
found peak 2 Val – Val – Tyr – Thr – Asp –(Cys)– Thr – Glu – Ser – Gly
found peak 3 Val – Val – Tyr – Thr.– Asp –(Cys)– Thr – Glu – Ser – Gly
found peak 4 Val – Val – Tyr – Thr – Asp –(Cys)– Thr – Glu – Ser – Gly
found peak 5 Val – Val – Tyr – Thr – Asp –(Cys)– Thr – Glu – Ser – Gly
found peak 6 Val – Val – Tyr – Thr – Asp –(Cys)– Thr – Glu – Ser – Gly


Cycle                                    15                           20
expected     Gln – Asn – Leu – Cys – Leu – Cys – Glu – Gly – Ser – Asn
found peak 1 Gln – Asn – Leu –(Cys)– Leu –(Cys)– Glu – Gly – Ser – Asn
found peak 2 Gln – Asn – Leu –(Cys)– Leu –(Cys)– Glu – Gly – Ser – Asn
found peak 3 Gln – Asn – Leu –(Cys)– Leu –(Cys)– Glu – Gly – Ser – Asn
found peak 4 Gln – Asn –
found peak 5 Gln – Asn –
found peak 6 Gln – Asn –
```

```
Cycle                                        25                                         30

expected       Val – Cys – Gly – Gln – Gly – Asn – Lys – Cys – Ile – Leu

found peak 1   Val –(Cys)– Gly – Gln – Gly – Asn – Lys –(Cys)– Ile – Leu

found peak 2   Val –(Cys)– Gly – Gln – Gly – Asn – Lys – ...

found peak 3   Val –(Cys)– Gly – Gln – Gly – Asn – Lys –(Cys)– Ile – ...


Cycle                                        35                                         40

expected       Gly – Ser – Asp – Gly – Glu – Lys – Asn – Gln – Cys – Val

found peak 1   Gly – Ser – Asp – Gly – Glu – Lys – Asn – Gln –(Cys)– Val


Cycle                                        45                                         50

expected       Thr – Gly – Glu – Gly – Thr – Pro – Lys – Pro – Gln – Ser

found peak 1   Thr – Gly – Glu – Gly – Thr – Pro – Lys – Pro – Gln – Ser


Cycle                                        55                                         60

expected       His – Asn – Asp – Gly – Asp – Phe – Glu – Glu – Ile – Pro

found peak 1   His – Asn – Asp – Gly – Asp – Phe – Glu – Glu – Ile – Pro


Cycle                                        65

expected       Glu – Glu – Tyr – Leu – Gln

found peak 1   Glu – Glu – Tyr – Leu – Gln

found peak 2   Glu – Glu – Tyr – Leu

found peak 3   Glu – Glu – Tyr

found peak 4   Glu – Glu – Tyr – (Leu/Gln)

found peak 5   Glu – Glu – Tyr – (Leu/Gln)

found peak 6   Glu – Glu – Tyr – (Leu/Gln)
```

D) Determination of the isoelectric point (IEP)

The hirudin compounds are subjected to standard isoelectric focussing (IEF) procedure (250-500 µg, Servalyte gel, pH 3 - 10), to titration-curve analysis (3.0 mg/9.3 cm, Servalyte gel, pH 3 - 10) and to chromato-focussing (see experimental conditions No. 4).

Experimental conditions No. 4:

Method
Ion exchange chromatography (IEC) on a strong cation exchanger stationary phase (pH-gradient elution)
Column Type: Mono-P (Pharmacia, Uppsala, Sweden)
Dimensions: 5.0 x 200 mm
Sample volume: 50 µl
Protein load/injection: 250 - 500 µg (5'000 - 10'000 µg/ml)
Flow rate: 1.0 ml/min
Counterpressure: ≃ 15 - 20 bar
Detection: at 280 nm (attn. 6)

21

Eluent A:
0.025 M Bis-Tris, pH 6.3

Eluent B:
H₂O/Pharmalyte 2.5-5 (Pharmacia) 50:1, pH 2.48

| Elution conditions: | t(min) | %B (v/v) |
|---|---|---|
| | 0 | 0 |
| | 6 | 0 |
| | 6.1 | 100 |
| | 36 | 100 |
| | 36.1 | 0 |
| | 70 | 0 |

Determination of the isoelectric point (IEP):
peaks are collected and the pH-value of the fractions is determined with standard pH meter

Results
The resulting IEP's are summarized in Table 5 and compared to the theoretically expected values, calculated by a computer programme.

Table 5

| Peak No. | Isoelectric point (pH) | | | |
|---|---|---|---|---|
| | calculated | detected by | | |
| | | IEF | Titr. curve | Chromat. focuss. |
| 1 | 3.9 | 3.8-3.9 | 3.9 | 3.8 |
| 2 | 3.9 | 3.8-3.9 n.a. | | n.a. |
| 3 | 3.9 | 3.8-3.9 n.a. | | n.a. |
| 4 | - | 3.9-4.1 n.a. | | n.a. |
| 5 | - | 3.9-4.1 n.a. | | n.a. |
| 6 | - | 3.6 n.a. | | n.a. |
| 7 | 3.9 | 3.7 n.a. | | n.a. |
| 8 | 3.9 | 3.8 n.a. | | 3.8 |

n.a.: not assayed.

Conclusions
Based on the presented data
- the compound of peak 1, representing the main product of the biosynthesis, is desulphatohirudin. It is identical to authentic desulphatohirudin [= peak 8] prepared from hirudin from the leech Hirudo medicinalis [= peak 7].
- The compounds of peaks 2, 3, 4, 5 and 6 are modified hirudin compounds found during biosynthesis (peaks 2 and 3) and purification (peaks 2 - 6) of recombinant desulphatohirudin.
- The compound of peak 2 is desulphatohirudin(1-64), lacking 1 amino acid at the C-terminus.
- The compound of peak 3 is desulphatohirudin(1-63), lacking 2 amino acids at the C-terminus.
- The compounds of peaks 4, 5 and 6 are biologically fully active analogs of desulphatohirudin with identical amino acid composition, N-terminal sequence (amino acids 1 - 12) and C-terminus (amino acids 61 - 65).

Example 15: Recovery of desulphatohirudin from S. cerevisiae cultured on a 50 l scale
The culture broth is mixed with Amberlite XAD-7 and is subjected to adsorption for about 4 hours at 25°C. The cells are separated from the resin in a column. After washing with 1M NaCl the resin is eluted with Tris buffer (50 mM, pH 7.0 - 8.5). The main fraction (30 l) is adjusted to pH 2.9 and is applied to a S-Sepharose column (Amicon PA, equilibrated with ammonium formiate buffer 25 mM, pH 2.9) having a bed volume of 2 l. After washing with ammonium formiate buffer (40 mM, pH 3.6) elution is done with ammonium formiate buffer (50 mM, pH 3.8). The main eluate fraction (10 l) is concentrated by means of a Filtron Minisette ultrafiltration system equipped with a Ω 3k membrane. A 0.5 l aliquot of the resulting clear protein solution is applied to a Bio-Gel P-6 fine column (Amicon GF equilibrated with 0.5 % acetic acid) having a bed volume of 1.5 l. Elution is done with 0.5 % acetic acid. The main eluate fraction (1 l) is concentrated by means of ultrafiltration and subsequently applied to a Q-Sepharose fast flow column (Amicon PA, equilibrated with ammonium formiate buffer 25 mM, pH 2.9) having a bed volume of 2 l. Elution is done with ammonium formiate buffer (50 mM, pH 4.2). The main eluate fraction is concentrated by means of ultrafiltration and is subsequently diafiltrated against water. The resulting clear aqueous solution is lyophilised. The solid consists of pure desulphatohirudin.

Deposition of microorganisms

The following microorganism strains were deposited at the Deutsche Sammlung von Mikroorganismen (DSM), Mascheroder Weg 1b, D-3300 Braunschweig (deposition dates and accession numbers given):
Saccharomyces cerevisiae H449: February 18, 1988, DSM 4413;
Escherichia coli JM109/pDP38: February 19, 1988, DSM 4414;
Escherichia coli JM109/pDP34: March 14, 1988, DSM 4473.

**Claims**

1. A yeast hybrid vector comprising (1) a desulphatohirudin expression cassette consisting of a constitutive yeast promoter operably linked to a first DNA sequence encoding a signal peptide linked in the proper reading frame to a second DNA sequence coding for a desulphatohirudin, and a DNA sequence containing yeast transcription termination signals, and (2) the complete two-micron DNA including intact REP1, REP2 and FLP genes, as well as intact ORI, STB, IR1 and IR2 sites.

2. A yeast hybrid vector according to claim 1 wherein the two-micron DNA additionally includes an intact D gene.

3. A yeast hybrid vector according to claim 1 wherein the constitutive yeast promoter is selected from the group consisting of a promoter of a gene encoding a glycolytic enzyme, ADHI promoter, TRPI promoter and the PH05 promoter which has been deprived of its upstream activation sites.

4. A yeast hybrid vector according to claim 1 wherein the first DNA sequence is selected from the group consisting of the hirudin signal sequence, the signal and prepro sequences of the yeast invertase, $\alpha$-factor, pheromone peptidase (KEX1), "killer toxin" and repressible acid phosphatase (PH05) genes and the glucoamylase signal sequence from Aspergillus awamori.

5. A yeast hybrid vector according to claim 1 wherein the second DNA sequence codes for a desulphatohirudin compound selected from the group consisting of desulphatohirudin variant HV1, HV2, HV2 (modified), PA and des(Val$_2$)-desulphatohirudin.

6. A yeast hybrid vector according to claim 1 comprising one or more selective genetic markers for yeast.

7. A yeast hybrid vector according to claim 1 comprising a selective genetic marker and an origin of replication for a bacterial host.

8. Method for the production of a yeast hybrid vector according to claim 1 comprising cleaving two-micron plasmid DNA with a restriction endonuclease such that the normal function of the REP1, REP2 and FLP genes and of the ORI, STB, IR1 and IR2 sites is maintained, linking the linearized plasmid obtained to the desulphatohirudin expression cassette and optionally to DNA segments containing one or more selective genetic markers for yeast and a selective genetic marker and a replication origin for a bacterial host, and recircularizing the hybrid vector obtained.

9. A yeast strain which is devoid of endogenous two-micron DNA and has been transformed with a hybrid vector comprising (1) a desulphatohirudin expression cassette consisting of a constitutive yeast promoter operably linked to a first DNA sequence encoding a signal peptide linked in the proper reading frame to a second DNA sequence coding for a desulphatohirudin, and a DNA sequence containing yeast transcription termination signals, and (2) the complete two-micron DNA including intact REP1, REP2 and FLP genes, as well as intact ORI, STB, IR1 and IR2 sites.

10. A Saccharomyces cerevisiae strain according to claim 9.

11. A yeast strain according to claim 9 transformed with a hybrid vector according to any one of claims 1 to 7.

12. Method for the production of a transformed yeast strain according to claim 9 comprising transforming a yeast strain which is devoid of endogenous two-micron plasmids with said hybrid vector.

13. Method for the production of desulphatohirudin compounds comprising culturing a yeast strain which is devoid of endogenous two-micron DNA and has been transformed with a hybrid vector comprising (1) a desulphatohirudin expression cassette consisting of a constitutive yeast promoter operably linked to a first DNA sequence encoding a signal peptide linked in the proper reading frame to a second DNA sequence coding for a desulphatohirudin, and a DNA sequence containing yeast transcription termination signals, and (2) the complete two-micron DNA including intact REP1, REP2 and FLP genes, as well as intact ORI, STB, IR1 and IR2 sites, and isolating said desulphatohirudins, and, if desired, separating a mixture of desulphatohirudin compounds obtained in the individual components.

14. Method according to claim 13 for the preparation of a desulphatohirudin compound selected from the group consisting of desulphatohirudin variant HV1, HV2, HV2 (modified), PA and des(Val$_2$)-desulphatohirudin.

23

15. Method according to claim 13 for the preparation of desulphatohirudin variant HV1.

16. Method according to claim 13 comprising culturing a yeast strain according to any one of claims 9 to 11.

17. Method according to claim 13 wherein the yeast strain has been transformed with a hybrid vector according to any one of claims 1 to 7.

**Claims for the following Contracting States: ES, GR**

1. Method for the production of a yeast hybrid vector comprising (1) a desulphatohirudin expression cassette consisting of a constitutive yeast promoter operably linked to a first DNA sequence encoding a signal peptide linked in the proper reading frame to a second DNA sequence coding for a desulphatohirudin, and a DNA sequence containing yeast transcription termination signals, and (2) the complete two-micron DNA including intact REP1, REP2 and FLP genes, as well as intact ORI, STB, IR1 and IR2 sites, which method comprises cleaving two-micron plasmid DNA with a restriction endonuclease such that the normal function of the REP1, REP2 and FLP genes and of the ORI, STB, IR1 and IR2 sites is maintained, linking the linearized plasmid obtained to the desulphatohirudin expression cassette and optionally to DNA segments containing one or more selective genetic markers for yeast and a selective genetic marker and a replication origin for a bacterial host, and recircularizing the hybrid vector obtained.

2. A method according to claim 1 wherein the two-micron DNA additionally includes an intact D gene.

3. A method according to claim 1 wherein the constitutive yeast promoter is selected from the group consisting of a promoter of a gene encoding a glycolytic enzyme, ADHI promoter, TRPI promoter and the PH05 promoter which has been deprived of its upstream activation sites.

4. A method according to claim 1 wherein the first DNA sequence is selected from the group consisting of the hirudin signal sequence, the signal and prepro sequences of the yeast invertase, α-factor, pheromone peptidase (KEX1), "killer toxin" and repressible acid phosphatase (PH05) genes and the glucoamylase signal sequence from Aspergillus awamori.

5. A method according to claim 1 wherein the second DNA sequence codes for a desulphatohirudin compound selected from the group consisting of desulphatohirudin variant HV1, HV2, HV2 (modified), PA and des(Val$_2$)-desulphatohirudin.

6. A method according to claim 1 wherein the yeast hybrid vector comprises one or more selective genetic markers for yeast.

7. A method according to claim 1 wherein the yeast hybrid vector comprises a selective genetic marker and an origin of replication for a bacterial host.

8. A yeast strain which is devoid of endogenous two-micron DNA and has been transformed with a hybrid vector comprising (1) a desulphatohirudin expression cassette consisting of a constitutive yeast promoter operably linked to a first DNA sequence encoding a signal peptide linked in the proper reading frame to a second DNA sequence coding for a desulphatohirudin, and a DNA sequence containing yeast transcription termination signals, and (2) the complete two-micron DNA including intact REP1, REP2 and FLP genes, as well as intact ORI, STB, IR1 and IR2 sites.

9. A Saccharomyces cerevisiae strain according to claim 8.

10. A yeast strain according to claim 8 transformed with a hybrid vector obtainable according to any one of claims 1 to 7.

11. Method for the production of a transformed yeast strain according to claim 8 comprising transforming a yeast strain which is devoid of endogenous two-micron plasmids with said hybrid vector.

12. Method for the production of desulphatohirudin compounds comprising culturing a yeast strain which is devoid of endogenous two-micron DNA and has been transformed with a hybrid vector comprising (1) a desulphatohirudin expression cassette consisting of a constitutive yeast promoter operably linked to a first DNA sequence encoding a signal peptide linked in the proper reading frame to a second DNA sequence coding for a desulphatohirudin, and a DNA sequence containing yeast transcription termination signals, and (2) the complete two-micron DNA including intact REP1, REP2 and FLP genes, as well as intact ORI, STB, IR1 and IR2 sites, and isolating said desulphatohirudins, and, if desired, separating a mixture of desulphatohirudin compounds obtained in the individual components.

13. Method according to claim 12 for the preparation of a desulphatohirudin compound selected from the group consisting of desulphatohirudin variant HV1, HV2, HV2 (modified), PA and des(Val$_2$)-desulpha-tohirudin.

14. Method according to claim 12 for the preparation of desulphatohirudin variant HV1.

15. Method according to claim 12 comprising culturing a yeast strain according to any one of claims 8 to 10.

16. Method according to claim 12 wherein the yeast strain has been transformed with a hybrid vector obtainable according to any one of claims 1 to 7.

___Fig. 1:___ In vitro synthesis of the PHO5 signal sequence - hirudin HV1 gene with preferred yeast codons

```
      EcoRI                                    PHO5 SS
      ----------1--------- _____3_____ --------
   5' AATTCAAAATGTTTAAATCTGTTGTTTATTCAATTTTAGCCGCTTCTTT
   3'     GTTTTACAAATTTAGACAACAAATAAGTTAAAATCGGCGAAGAAA
      ------------2-------------------  _____4_____

          ------>YHIR
   --5--------- _____7_____  ----------------
   GGCCAATGCAGTTGTTTACACCGACTGTACCGAATCTGGTCAAAACTTGT
   CCGGTTACGTCAACAAATGTGGCTGACATGGCTTAGACCAGTTTTGAACA
   __ ------------6--------- _____8_____

   -9---------- _____11_____  ---------------13-
   GTTTGTGTGAAGGTTCTAACGTTTGTGGTCAAGGTAACAAGTGTATCTTG
   CAAACACACTTCCAAGATTGCAAACACCAGTTCCATTGTTCACATAGAAC
   _ ------------10-------------|_____12_____

   --------- _____15_____  ---------------17
   GGTTCTGACGGTGAAAAGAACCAATGTGTTACCGGTGAAGGTACCCCAAA
   CCAAGACTGCCACTTTTCTTGGTTACACAATGGCCACTTCCATGGGGTTT
   _ ------------14--------- _____16_____ -----

   ------------ _____19_____  -----------
   GCCACAATCTCACAACGACGGTGACTTCGAAGAAATCCCAGAAGAATACT
   CGGTGTTAGAGTGTTGCTGCCACTGAAGCTTCTTTAGGGTCTTCTTATGA
   -----18---------------------- _____20__

   -21-----
   TGCAATAG      3'
   ACGTTATCCTAG 5'
```

   _____
         BamHI

**Fig. 2:** CONSTRUCTION OF PLASMID pDP33

Fig. 3: Construction of plasmids pDP34 and pDP38

EP 0 340 170 A2

*Fig. 4:* Construction of plasmid pDP34/GAPFL-YHIR

*Fig. 5:* Construction of plasmid pDP34/PH05(-173)-YHIR

Fig. 6: Construction of plasmid pDP92

**Fig. 7:** Plasmid pDP96

EP 0 340 170 A2